(19) Europäisches Patentamt
European Patent Office
Office européen des brevets

(11) Numéro de publication: **0 265 305 B1**

(12) # FASCICULE DE BREVET EUROPEEN

(45) Date de publication de fascicule du brevet: **12.06.91**

(51) Int. Cl.[5]: **C07D 231/22**, C07D 231/20, C07D 417/04, C07D 401/04, C07D 403/04, A01N 43/56

(21) Numéro de dépôt: **87402084.5**

(22) Date de dépôt: **18.09.87**

Le dossier contient des informations techniques présentées postérieurement au dépôt de la demande et ne figurant pas dans le présent fascicule.

(54) **Nouveaux dérivés de la 5-pyrazolone, leur procédé de préparation, leur utilisation comme herbicides et les compositions les renfermant.**

(30) Priorité: **18.09.86 FR 8613050**

(43) Date de publication de la demande: **27.04.88 Bulletin 88/17**

(45) Mention de la délivrance du brevet: **12.06.91 Bulletin 91/24**

(84) Etats contractants désignés: **CH DE FR GB IT LI NL**

(56) Documents cités:
**EP-A- 0 165 448**

**CHEMICAL ABSTRACTS, vol. 85, no. 1, 5 juillet 1976, page 453, abrégé no. 5618x, Columbus, Ohio, US; & JP-A-75 116 473**

**CHEMICAL ABSTRACTS, vol. 85, no. 21, 22 novembre 1976, pages 550,551, abrégé no. 160082m, Columbus, Ohio, US; & JP-A-76 19 768**

(73) Titulaire: **ROUSSEL-UCLAF**
**35, boulevard des Invalides**
**F-75007 Paris(FR)**

(72) Inventeur: **Tesssier, Jean**
**30, rue Jean Moulin**
**F-94300 Vincennes(FR)**
Inventeur: **Girault, Pierre**
**4, rue des Abbesses**
**F-75018 Paris(FR)**

(74) Mandataire: **Tonnellier, Marie-José et al**
**Département des Brevets ROUSSEL UCLAF**
**B.P. no 9**
**F-93230 Romainville(FR)**

**EP 0 265 305 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés de la 5-pyrazolone, leur procédé de préparation, leur utilisation comme herbicides et les compositions les renfermant.

L'invention a pour objet les composés de formule (I) :

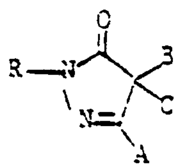

(I)

dans laquelle,

- R représente ou bien un radical phényle ou naphtyle, ou bien un radical thiazolyle, pyridinyle ou pyrimidyle éventuellement substitué par un radical phényle, ou bien un radical

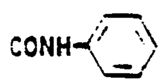

ou bien un radical benzyle, le radical R étant éventuellement substitué par un ou plusieurs radicaux choisis dans le groupe constitué par les atomes de fluor, de chlore, de brome, le radical $CF_3$, les radicaux alcoyles linéaires ou ramifiés, saturés ou insaturés renfermant jusqu'à 8 atomes de carbone et le radical OZ dans lequel Z représente un radical alcoyle linéaire ou ramifié, saturé ou insaturé renfermant jusqu'à 8 atomes de carbone ou un radical acyle renfermant jusqu'à 6 atomes de carbone,
- A représente un atome d'halogène, un radical $CF_3$ ou un radical C≡N,
- B et C identiques ou différents représentent soit un atome d'hydrogène, soit un atome d'halogène, soit un radical hydroxy, soit un radical OR' dans lequel R' représente un radical alcoyle linéaire ou ramifié, saturé ou insaturé renfermant jusqu'à 8 atomes de carbone, un radical acyle renfermant jusqu'à 6 atomes de carbone, un radical alkylsulfonyl renfermant jusqu'à 8 atomes de carbone ou un radical benzylsulfonyl, soit un radical benzyle éventuellement substitué par un ou plusieur radicaux choisis dans le groupe constitué par les atomes de fluor, de chlore, de brome, le radical $CF_3$ et les radicaux alcoyles linéaires ou ramifiés, saturés ou insaturés renfermant jusqu'à 8 atomes de carbone, soit un radical alcoyle renfermant de 1 à 8 atomes de carbone linéaire ou ramifié, saturé ou insaturé, éventuellement interrompu par un ou plusieurs atomes d'oxygène et éventuellement substitué par un ou plusieurs atomes d'halogène, étant entendu que B et C ne peuvent pas représenter en même temps soit un atome de fluor, soit un radical hydroxy, et que B et C ne peuvent pas représenter en même temps un atome d'hydrogène lorsque A représente un radical CN et R représente un radical 3-chlorophényle.

Lorsque R est substitué par un radical alcoyle, il s'agit de préférence d'un radical méthyle, éthyle, n-propyle, isopropyle, n-butyle, isobutyle, terbutyle, n-pentyle, héxyle ou du radical allyle ou 2-méthyl allyle.

Lorsque A, B ou C représente un atome d'halogène, il s'agit de préférence d'un atome de brome, de chlore ou d'iode.

Lorsque B, C, Z ou R' représente un radical alcoyle, il s'agit de préférence d'un radical méthyle, éthyle, n-propyle, isopropyle, n-butyle ou terbutyle, n-pentyle ou n-hexyle.

Lorsque B et C sont substitués par un ou plusieurs atomes d'halogène, il s'agit de préférence d'un ou plusieurs atomes de chlore ou de brome.

Lorsque Z ou R' représente un radical acyle, il s'agit de préférence d'un radical acétyle ou propionyle.

Lorsque R' représente un radical alkylsulfonyle, il s'agit de préférence du radical méthylsulfonyle, éthylsulfonyle.

Parmi les composés préférés de l'invention, on peut citer les composés de formule I dans lesquels R représente un radical phényle éventuellement substitué par un ou plusieurs atomes de chlore ou de brome, ceux dans lesquels A représente un atome de chlore, un radical $CF_3$ ou un radical C≡N, ainsi que ceux dans lesquels les radicaux B et C identiques ou différents représentent un atome d'hydrogène, un atome de chlore ou de brome, un radical alcoyle saturé linéaire ou ramifié renfermant jusqu'à 6 atomes de carbone ou un radical allyle.

L'invention a tout particulièrement pour objet les composés dont la préparation est donnée plus loin

dans la partie expérimentale. Parmi ces composés, on peut citer notamment le produit répondant à la formule (I) dont le nom suit :

- la 1-(3,4-dichlorophényl) 3,4-dichloro 4-(2-méthylpropyl) 5-pyrazolone, ainsi que les produits répondant à la formule (I) dont les noms suivent :
- la 1-(3-chloro 4-bromophényl) 3-trifluorométhyl 4-chloro 4-méthyl 5-pyrazolone,
- la 1-(3,5-dichlorophényl) 3,4-dichloro 4-méthyl 5-pyrazolone,
- la 1-(3,4-dichlorophényl) 3,4-dichloro 4-n-propyle 5-pyrazolone.

L'invention a également pour objet un procédé de préparation des composés de formule (I) caractérisé en ce que l'on soumet un composé de formule (II) :

$H_2N\text{-}NH\text{-}R$ (II)

dans laquelle R conserve la même signification que précédemment à l'action d'un composé de formule (III) :

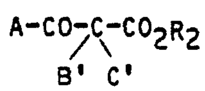

(III)

dans laquelle A conserve sa signification précédente et B′ et C′ ayant la signification indiquée pour B et C à l'exception du radical OH ou OR′ et $R_2$ représente un radical alcoyle renfermant jusqu'à 8 atomes de carbone pour obtenir le composé de formule ($I_A$) :

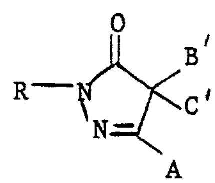

($I_A$)

dans laquelle R, A , B′ et C′ ont la signification indiquée ci-dessus que l'on soumet éventuellement si l'un ou l'autre (ou l'un et l'autre) des radicaux B′ et C′ représente un atome d'hydrogène, soit à l'action d'un agent d'halogénation pour obtenir le dérivé de formule (I) correspondant dans lequel B′ ou C′ ou B′ et C′ représentent un atome d'halogène, soit à l'action d'un agent d'oxydation pour obtenir le dérivé de formule (I) correspondant dans lequel B′ ou C′ représente un radical hydroxy, produit que l'on soumet, si désiré, soit à l'action d'un agent capable d'introduire un radical alkyle, acyle, benzylsulfonyle, alkylsulfonyle, soit à l'action d'un agent d'halogénation pour obtenir le produit de formule (I) correspondant.

Dans un mode de réalisation préféré des composés de l'invention

- la réaction entre le composé II et le composé III a lieu au sein d'un milieu alcoolique et dans le cas où R représente le radical

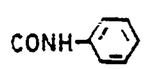

on traite le produit obtenu par action du produit II sur le produit III par une base comme l'ammoniaque et obtient le produit cyclisé I
- l'agent d'halogénation utilisé est l'halogène lui-même à l'état moléculaire.

L'invention a également pour objet une variante du procédé de préparation décrit ci-dessus caractérisée en ce que l'on soumet le composé de formule (II) :

$H_2N\text{-}NHR$ (II)

dans laquelle R conserve sa signification précédente, à l'action du composé de formule (IV) :

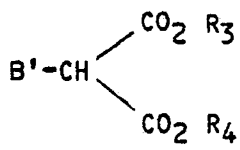

(IV)

dans laquelle B′ conserve sa signification précédente, $R_3$ et $R_4$ identiques ou différents représentent un radical alcoyle renfermant jusqu'à 6 atomes de carbone pour obtenir le composé de formule (V) :

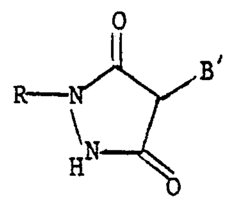

(V)

que l'on soumet à l'action d'un agent de chloruration pour obtenir

- si B′ ne représente pas un atome d'hydrogène, le composé de formule ($I_B$) ou ($I_C$) :

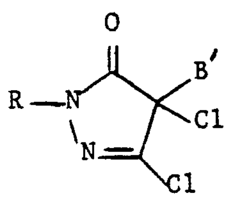

ou

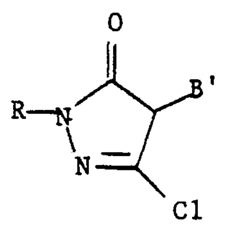

($I_B$) ($I_C$)

- et si B′ représente un atome d'hydrogène le composé de formule ($I_D$) :

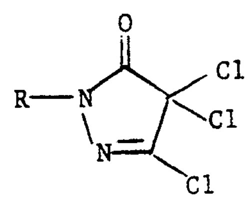

puis soumet si désiré le produit de formule ($I_C$) à l'action du chlore pour obtenir le produit de formule ($I_D$) correspondant ou bien à l'action d'un agent d'oxydation pour obtenir le produit un formule ($I_E$) :

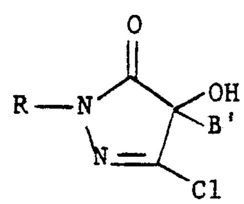

($I_E$)

que l'on soumet si désiré soit à l'action d'un agent capable d'introduire un radical alkyle, acyle, alkylsulfonyle ou benzylsulfonyle, soit à l'action d'un agent d'halogénation pour obtenir le produit de formule (I) correspondant.

Dans un mode de réalisation préféré du procédé de l'invention : la réaction entre le composé de formule II et le composé de formule IV a lieu à une température comprise entre 100 et 200¤C.

L'agent de chloruration utilisé est un mélange de pentachlorure de phosphore et d'oxychlorure de phosphore.

L'invention a également pour objet une variante du procédé ci-dessus caractérisée en ce que l'on

soumet un composé de formule (VI) :

$RNH_2$ (VI)

dans laquelle R conserve la même signification que précédemment, à l'action d'un agent de diazotation, pour obtenir le sel de diazonium correspondant que l'on soumet à l'action du composé de formule (VII) :

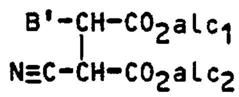

(VII)

dans laquelle B′ conserve la même signification que précédemment, $alc_1$ et $alc_2$, identiques ou différents représentent un radical alcoyle renfermant jusqu'à 8 atomes de carbone pour obtenir le composé de formule ($I_F$) :

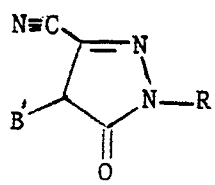

($I_F$)

que l'on soumet éventuellement soit à l'action d'un agent d'halogénation pour obtenir si B′ représente un atome d'hydrogène, le composé dihalogéné de formule ($I_G$) :

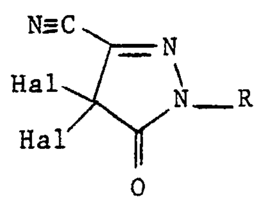

($I_G$)

et si B′ ne représente pas un atome d'hydrogène, le composé monohalogéné correspondant ($I_H$) :

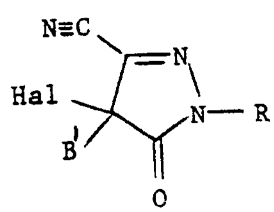

($I_H$)

soit à l'action d'un agent d'oxydation pour obtenir un produit de formule ($I_I$) :

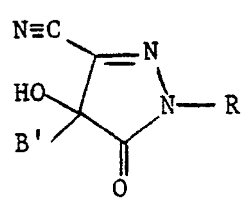

($I_I$)

que l'on soumet si désiré soit à l'action d'un agent capable d'introduire un radical alkyle, acyle, alkylsulfonyle ou benzylsulfonyle soit à l'action d'un agent d'halogénation pour obtenir le produit de formule (I)

correspondant.

Dans un mode de réalisation préféré l'agent de diazotation utilisé est un nitrite alcalin comme le nitrite de sodium ou de potassium ou un nitrite d'alcoyle.

L'invention a aussi pour objet les compositions pesticides notamment herbicides caractérisées en ce qu'elles contiennent comme matière active au moins l'un des composés tels que définis précédemment.

L'invention a notamment pour objet les compositions herbicides utilisées pour la culture des céréales comme le blé, l'orge, l'avoine, le seigle et le maïs ou pour la culture du riz, du coton et du soja, caractérisées en ce qu'elles contiennent comme matière active au moins l'un des composés tels que définis précédemment.

L'invention a aussi pour objet l'application des compositions telles que définies précédemment au désherbage sélectif des céréales comme le blé, l'orge, l'avoine, le seigle et le maïs, ou du riz, du coton et du soja.

Les compositions selon l'invention peuvent se présenter sous forme de poudres, granulés, suspensions, émulsions, solutions contenant le principe actif, par exemple, en mélange avec un véhicule et/ou un agent tensio-actif anionique, cationique ou non ionique assurant entre autres, une dispersion uniforme des substances de la composition. Le véhicule utilisé peut être un liquide, tel que l'eau, l'alcool, les hydrocarbures ou autres solvants organiques, une huile minérale, animale ou végétale, ou une poudre, telle que le talc, les argiles, les silicates, le kieselguhr.

Les compositions solides, présentées sous forme de poudre pour poudrage, de poudres mouillables ou de granulés, peuvent être préparées par broyage du composé actif avec un solide inerte ou par imprégnation d'un support solide avec une solution du principe actif dans un solvant que l'on évapore ensuite.

Outre un véhicule et/ou un agent tensio-actif, ces compositions contiennent, comme principe actif, un ou plusieurs composés de formule I, ainsi qu'éventuellement d'autres pesticides et des substances présentant des propriétés influant sur la croissance des plantes.

Les compositions de l'invention sont, bien entendu, appliquées à des doses suffisantes pour exercer leurs activités herbicides. Les doses de matière active dans les compositions varient notamment en fonction des végétaux à détruire, de la nature du terrain, des conditions atmosphériques et de l'état d'avancement de la végétation à détruire.

Les compositions herbicides selon l'invention contiennent en général de 10 à 80% en poids et, de préférence, de 10 à 50% en poids de matière active.

Lors de l'utilisation des composés I comme herbicide, les quantités de substance active appliquées varient en pré-levée de préference entre 0,1 et 5 Kg/ha et en post-levée, de préference entre 0,5 et 2 Kg/ha.

Les exemples suivant illustrent l'invention sans toutefois la limiter.

**Exemple 1 : 1-(4-bromo 3-chlorophényl) 3-trifluorométhyl 4-allyl 4-chloro 5-pyrazolone (mode opératoire I).**

**Stade A :** 1-(4-bromo 3-chlorophényl) 3-trifluorométhyl 4-allyl 5-pyrazolone.

On chauffe au reflux pendant 19 heures un mélange de 12,1g de 2-allyl 4,4,4-trifluoroacétoacétate d'éthyle, 10 g de 4-bromo 3-chlorophénylhydrazine et 40 cm3 d'éthanol. On ajoute 20 cm3 d'eau et 20 cm3 d'acide acétique et chauffe au reflux pendant 22 heures. On laisse refroidir. On extrait avec de l'éther, lave avec une solution aqueuse saturée de bicarbonate de sodium puis avec de l'eau. On sèche sur sulfate de sodium anhydre et concentre sous vide. On obtient 18,7g de produit que l'on chromatographie sur silice en éluant par le mélange hexane-éther éthylique 1-1. On obtient 5,30g de produit recherché fondant à 117¤C après reprise par du pentane.

**Stade B :** 1-(4-bromo 3-chlorophényl) 3-trifluorométhyl 4-allyl 4-chloro 5-pyrazolone.

On refroidit à 0¤C, un mélange de 4,06g de produit préparé au stade A et 150 cm3 de tétrachlorure de carbone. On ajoute 17,2 cm3 de chlore en solution dans le tétrachlorure de carbone (1,55 mole par litre). Quand la réaction est terminée, on lave avec une solution saturée de bicarbonate de soude à l'eau, sèche, traite au charbon actif et maintient sous agitation pendant 15 heures. On filtre et concentre, on obtient 51 g de produit attendu.

**Exemple 2 : 1-(phénylcarbamoyl) 3-trifluorométhyl 5-pyrazolone (mode opératoire 2).**

**Stade A :** 4,4,4-trifluoro 3-(phényl semi carbazono) butyrate d'éthyle.

On agite et chauffe au reflux pendant 5 heures 15,1g de 4-phényle semi carbazide, 18,4g de trifluoroacétylacétate d'éthyle, 80 cm3 d'éthanol et 10 cm3 d'acide acétique. On laisse refroidir. On glace, essore, lave et sèche les cristaux. On obtient 14g du produit brut que l'on purifie par dissolution dans le chlorure de méthylène, filtration de l'insoluble et distillation à sec sous pression réduite. On sèche et obtient 11,2g de produit recherché fondant à 162-163¤C.

**Stade B :** 1-(phénylcarbamoyl) 3-trifluorométhyl 5-pyrazolone.
On agite pendant 3 heures 10 g de 4,4,4-trifluoro 3-(phényl semi carbazono) butyrate d'éthyle et 200 cm3 d'ammoniaque. On ajoute 200 cm3 d'eau, filtre l'insoluble et lave à l'eau. On réunit les filtrats, les acidifie avec de l'acide chlorhydrique dilué, essore le précipité formé, lave, sèche et obtient 7,20g de produit recherché fondant à 109-110¤C.

**Exemple 3 : 1-(3,5-dichlorophényl) 3,4-dichloro 4-méthyl 5-pyrazolone (mode opératoire 3).**

**Stade A :** 1-(3,5-dichlorophényl) 4-méthyl pyrazolidine 3,5-dione.
On porte 5 heures à 180¤C une solution renfermant 17,55g de 3,4-dichlorophénylhydrazine et 35g de méthylmalonate de diéthyle en distillant. On élimine les produits distillés. On refroidit et reprend au chlorure de méthylène. On extrait la phase organique à la soude diluée 2N et lave au chlorure de méthyle. On traite au charbon actif. On acidifie à pH 1 avec de l'acide chlorhydrique. On essore le précipité obtenu, lave à l'eau et sèche sous pression réduite. On obtient 16,9g du produit recherché fondant à 95-100¤C.

**Stade B :** 1-(3,5-dichlorophényl) 3,4-dichloro 4-méthyl 5-pyrazolone.
On porte au reflux pendant 16 heures 21,6g de produit préparé au stade A, 90 cm3 d'oxychlorure de phosphore et 56g de pentachlorure de phosphore. On refroidit et verse sur de la glace. On essore le précipité obtenu, lave à l'eau et sèche. On obtient 19,3g de produit brut que l'on chromatographie sur silice en éluant par le mélange chlorure de méthylène-hexane 3-7. On obtient 4,35g du produit recherché fondant à 77¤C.

**Exemple 4 : 1-(3,4-dichlorophényl) 3-cyano 4-chloro 4-méthyl 5-pyrazolone (mode opératoire 4).**

**Stade A :** 1-(3,4-dichlorophényl) 3-cyano 4-méthyl 5-pyrazolone.
On introduit à 0¤C sous agitation 10g de nitrite de sodium et 30 cm3 d'eau dans un mélange de 22,7g de 3,4-dichloroaniline, 100 cm3 d'eau et 30 cm3 d'acide chlorhydrique concentré. On agite 15 minutes à 0¤C et obtient une solution que l'on verse à 15-20¤C dans une solution renfermant 33g de 2-cyano 3-méthyl succinate d'éthyle et 600 cm3 de pyridine. On agite 2 heures à 20¤C. On introduit simultanément 186 cm3 de triéthylamine d'une part et 310 cm3 d'une solution aqueuse de soude à 2%. On agite ensuite 3 heures à 20¤C. On verse dans 1 500 cm3 d'eau glacée et sous agitation amène à pH 3-4 à l'aide d'acide chlorhydrique concentré. On essore, lave à l'eau et sèche sous pression réduite et obtient 38g de produit recherché brut que l'on recristallise dans l'acétonitrile et sèche le produit solvaté et obtient 27g de produit recherché fondant à 210¤C.

**Stade B :** 1-(3,4-dichlorophényl) 3-cyano 4-chloro 4-méthyl 5-pyrazolone.
On introduit à 20¤C environ 41,5 cm3 d'une solution de chlore à 8,1% dans le tétrachlorure de carbone dans une solution de 12,7g de 1-(3,4-dichlorophényl) 3-cyano 4-méthyl 5-pyrazolone dans 100 cm3 de chloroforme. On agite pendant 1 heure à 20¤C et obtient après distillation des solvants, sous pression réduite 17g d'une huile jaune que l'on purifie par chromatographie sur silice en éluant par le mélange chlorure de méthylènehexane 8-2. On obtient 12,2g de produit recherché fondant à 68-69¤C.

Exemple 5 :

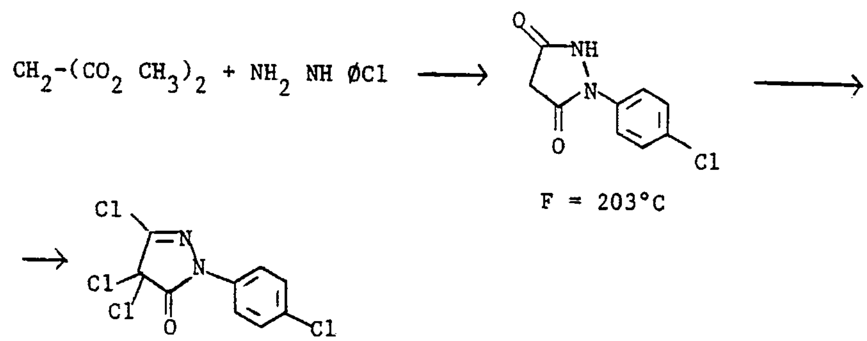

F = 203°C

F = 67°C

mode opératoire 3

Exemple 6 :

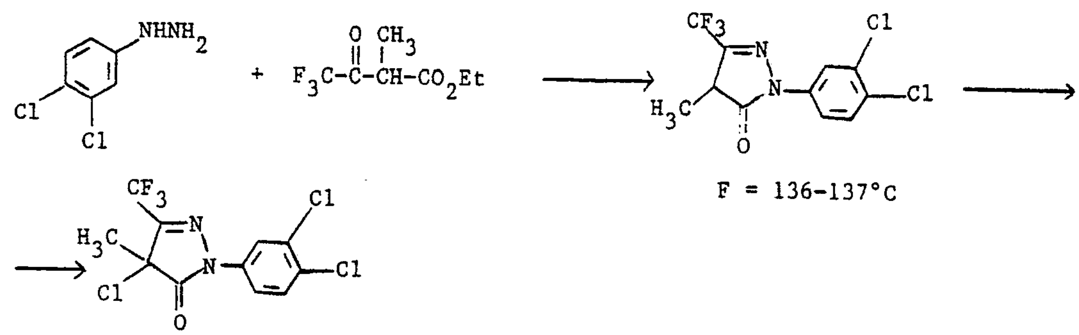

F = 136-137°C

F = 62-63°C

mode opératoire 1

Exemple 7 :

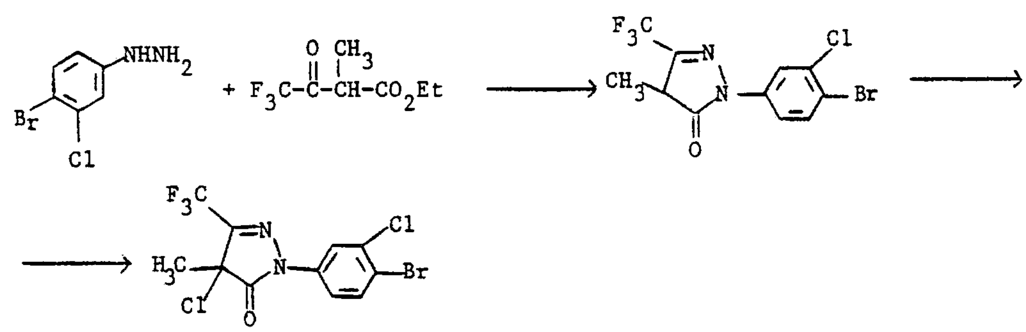

F = 66-67°C

mode opératoire 1

Exemple 8 :

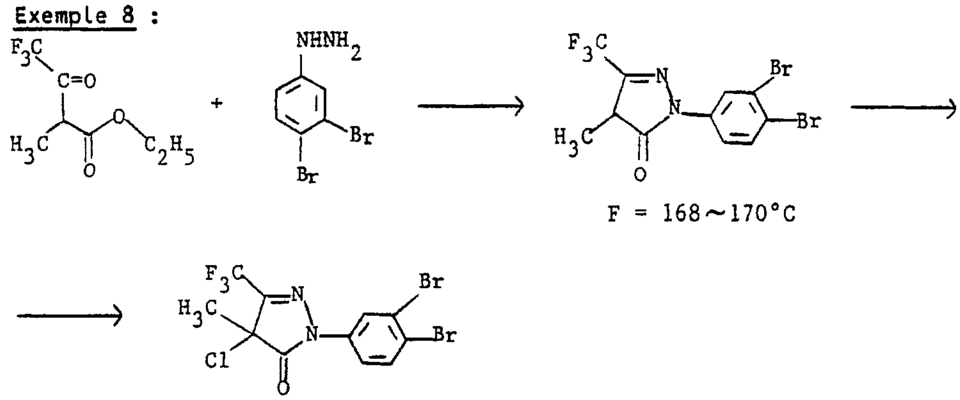


F = 62-64°C

mode opératoire 1

Exemple 9 :

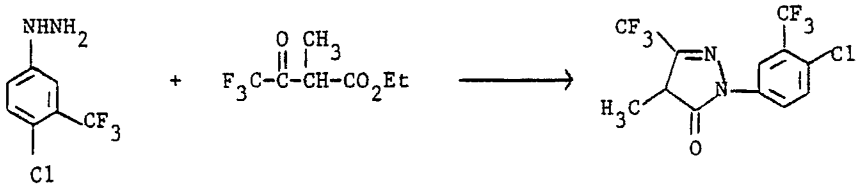

F = 159-160°C

mode opératoire 1

Exemple 10 :

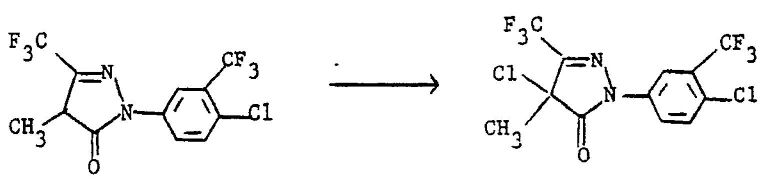

F = 64°C

mode opératoire 1

Exemple 11 :

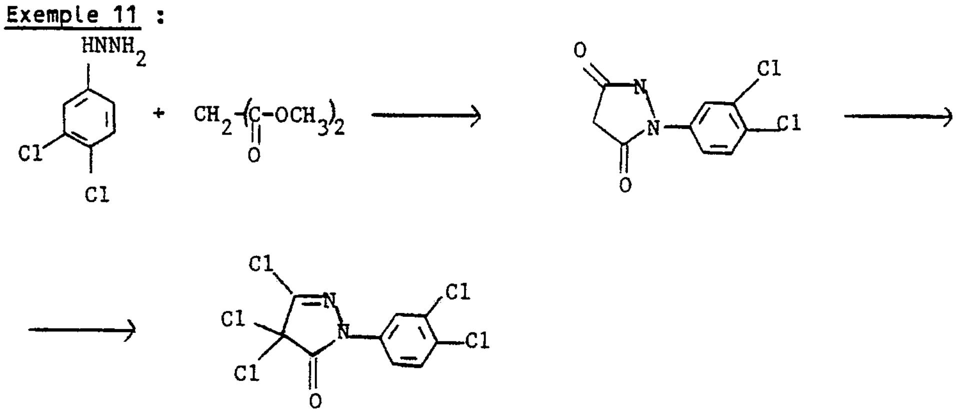

mode opératoire 3

Exemple 12 :

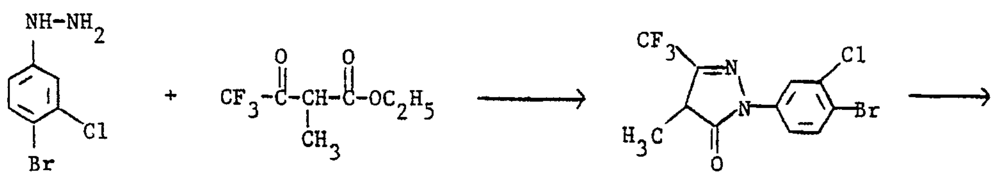

F = 160°C

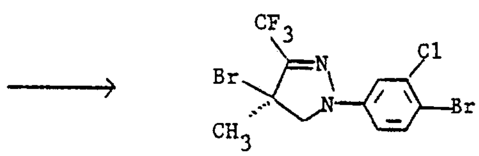

F = 93 ~ 94°C

mode opératoire 1

Exemple 13 :

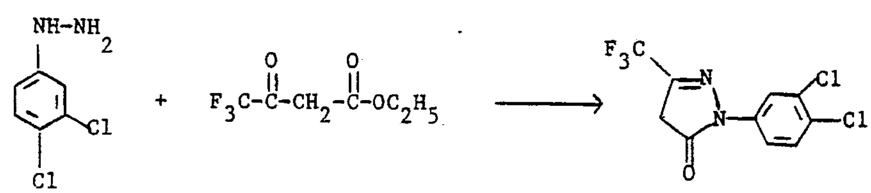

F = 220°C

mode opératoire 1

Exemple 14 :

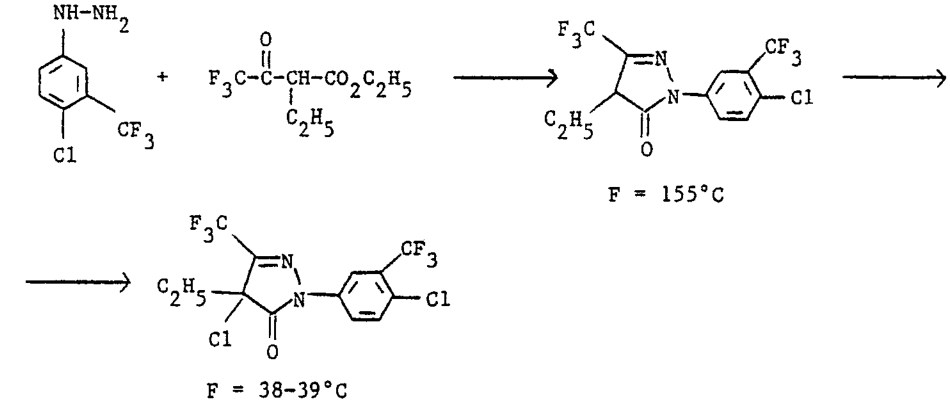


mode opératoire 1

Exemple 15 :

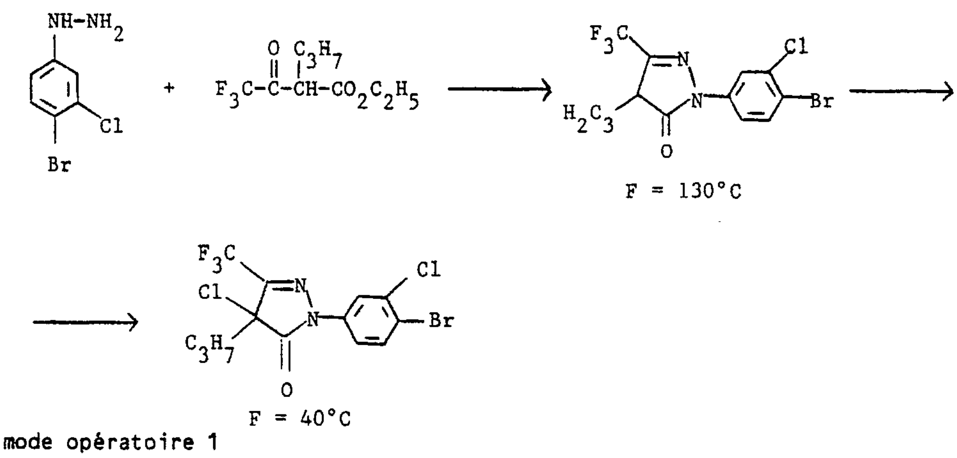


mode opératoire 1

Exemple 16 :

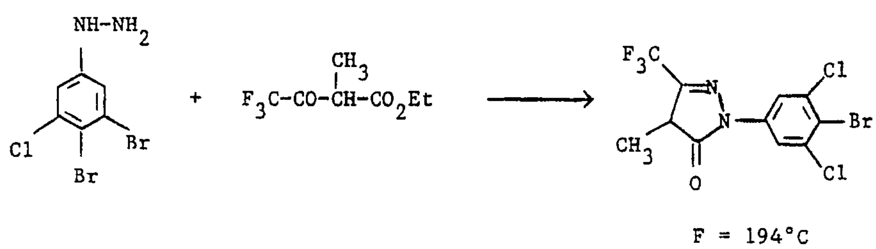


mode opératoire 1

Exemple 17 :

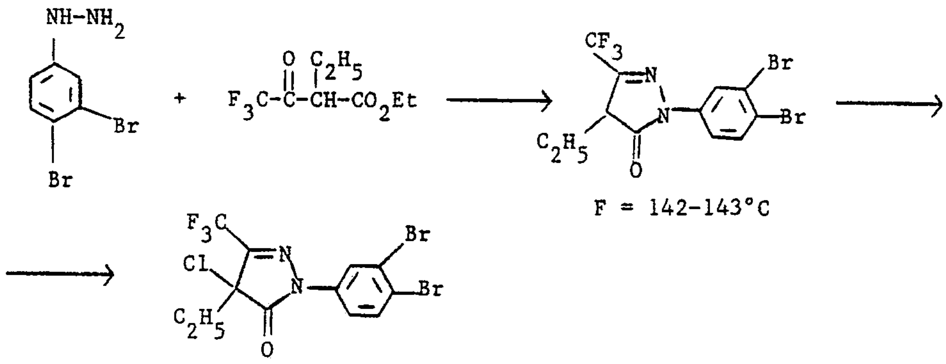

F = 142-143°C

F = 90~91°C

mode opératoire 1

Exemple 18 :

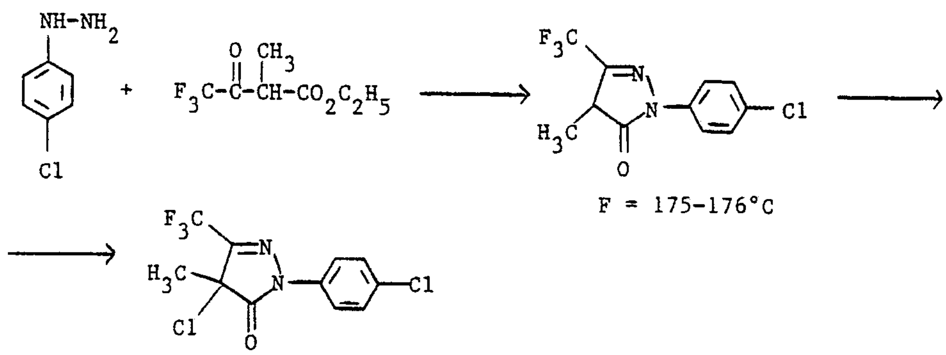

F = 175-176°C

F = 67-68°C

mode opératoire 1

Exemple 19 :

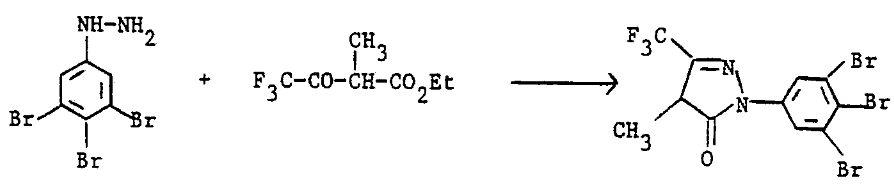

F = 202°C

mode opératoire 1

Exemple 20 :

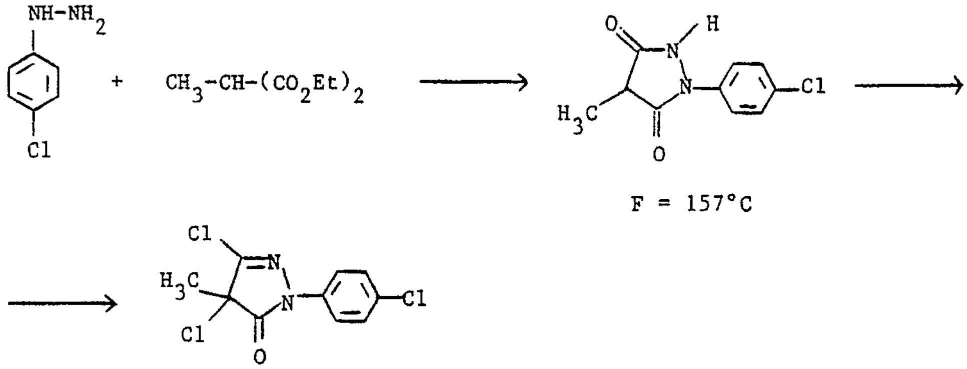

F = 157°C

F = 45°C

mode opératoire 3

Exemple 21 :

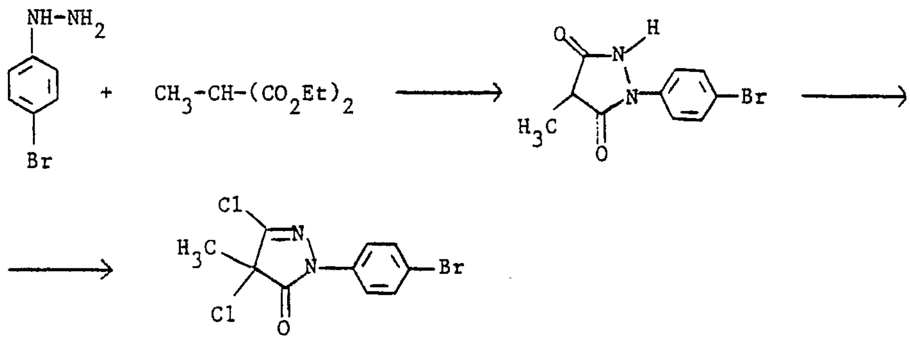

F = 40°C

mode opératoire 3

Exemple 22 :

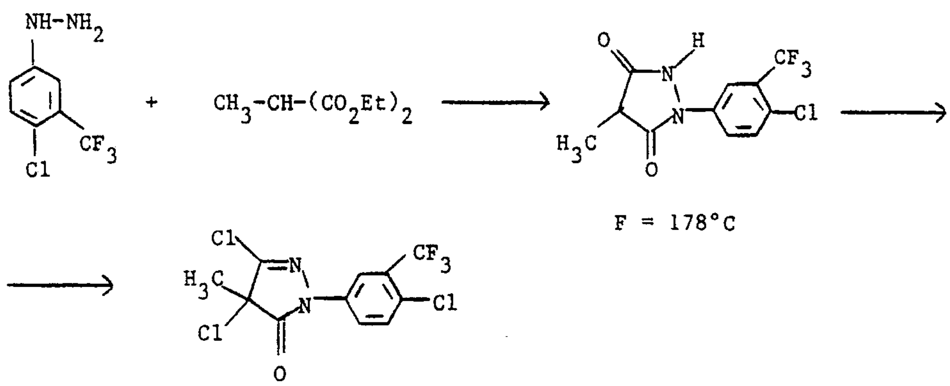

F = 178°C

F = 57°C

mode opératoire 3

Exemple 23 :

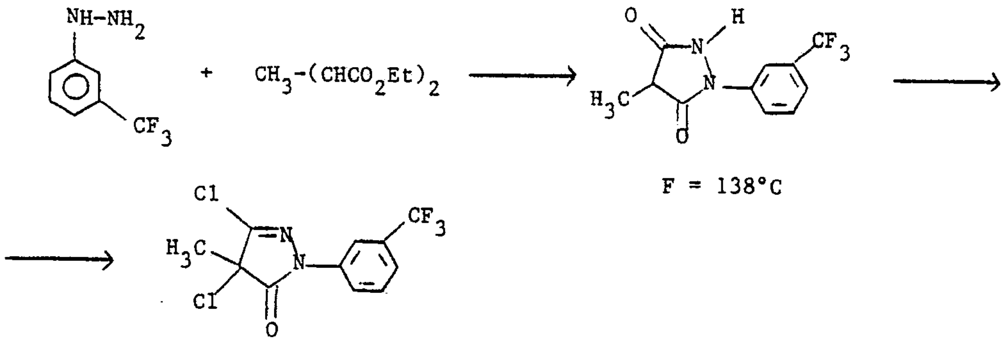


mode opératoire 3

Exemple 24 :

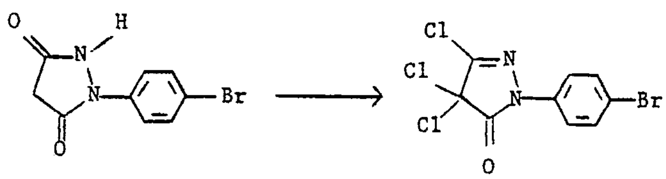

mode opératoire 3

Exemple 25 :

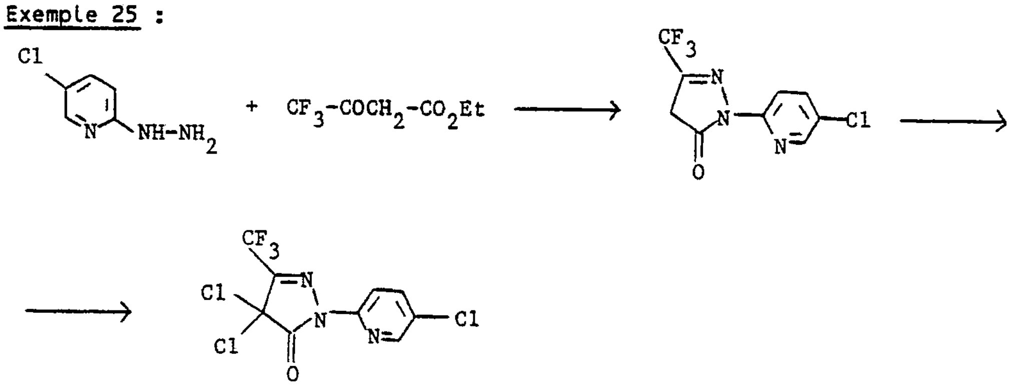

F = 108°C

mode opératoire 1

Exemple 26 :

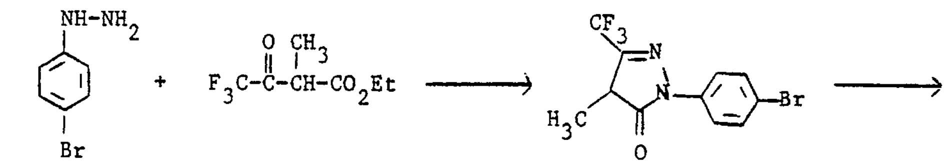

F = 160 ~ 161°C

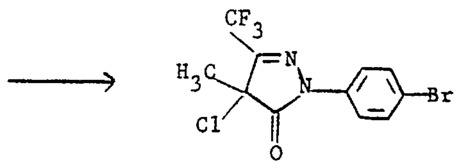

F = 75 ~ 76°C

mode opératoire 1

Exemple 27 :

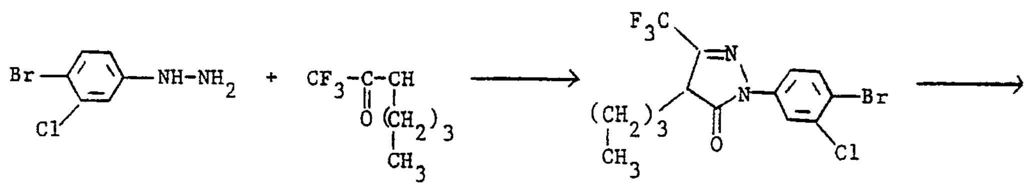

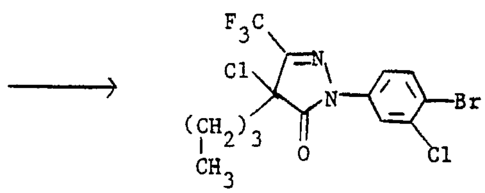

mode opératoire 1

Exemple 28 :

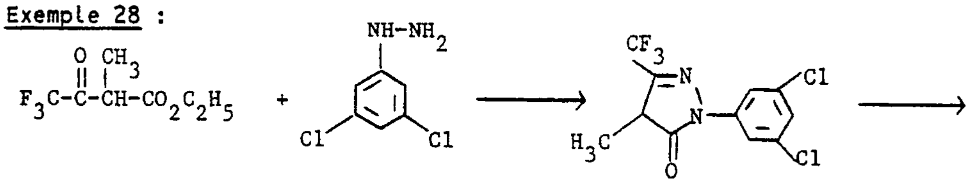

F = 173-174°C

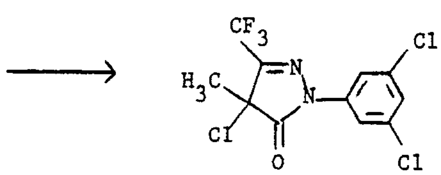

F = 90-91°C

mode opératoire 1

Exemple 29 :

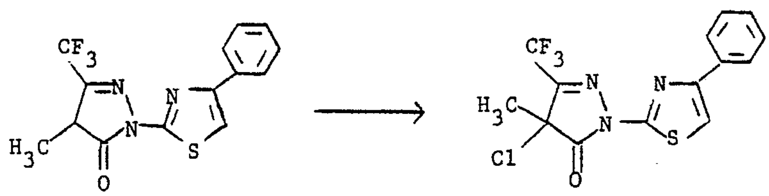

F = 80°C

mode opératoire 1

Exemple 30 :

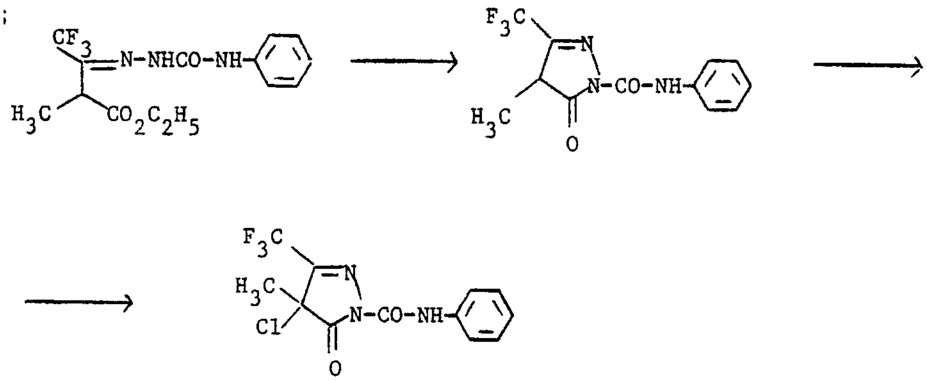

F = 142~143°C

mode opératoire 2

Exemple 31 :

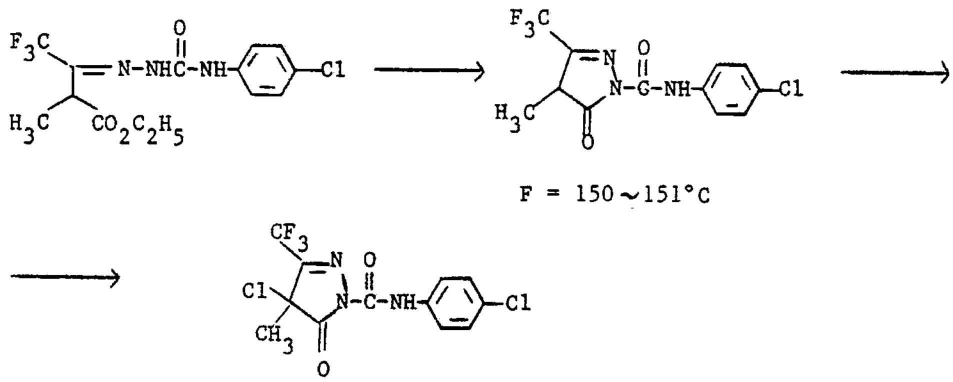

F = 150~151°C

F = 114~115°C

mode opératoire 2

Exemple 32 :

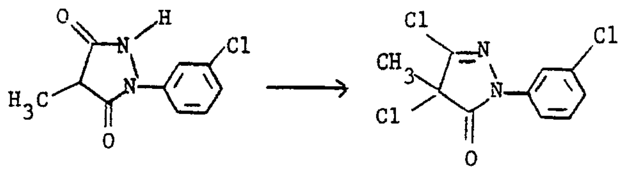

mode opératoire 3

Exemple 33 :

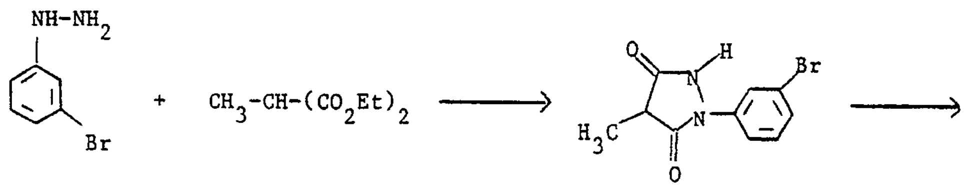

F = 100°C

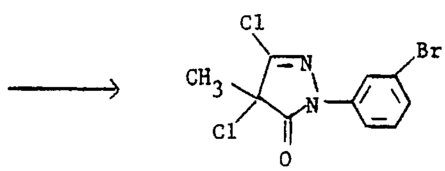

mode opératoire 3

Exemple 34 :

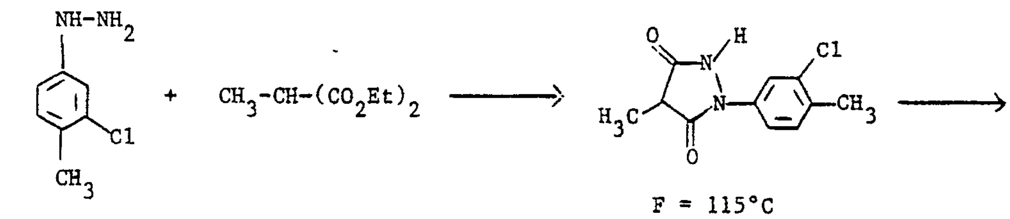

F = 115°C

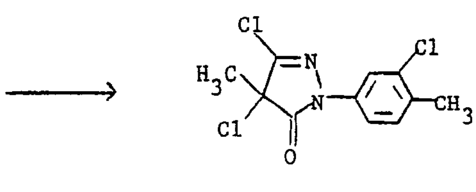

F = 59°C

mode opératoire 3

Exemple 35 :

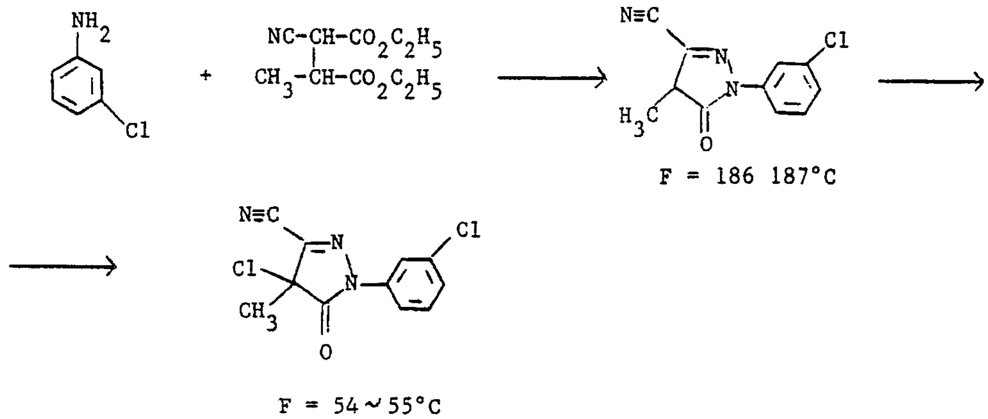


mode opératoire 4

Exemple 36 :

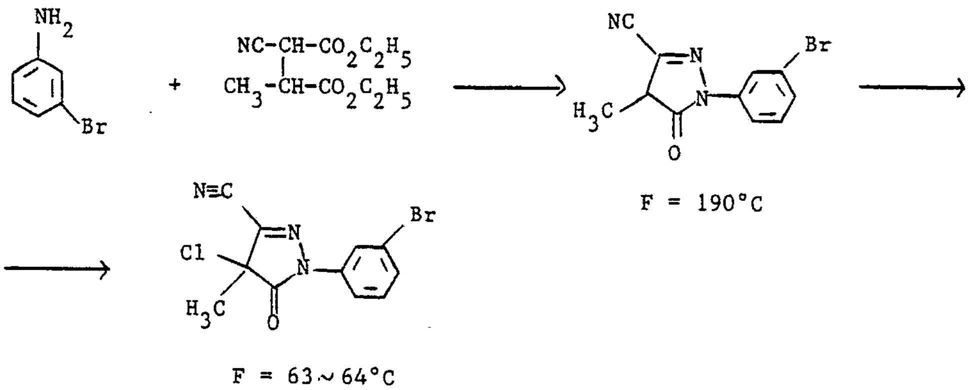


mode opératoire 4

Exemple 37 :

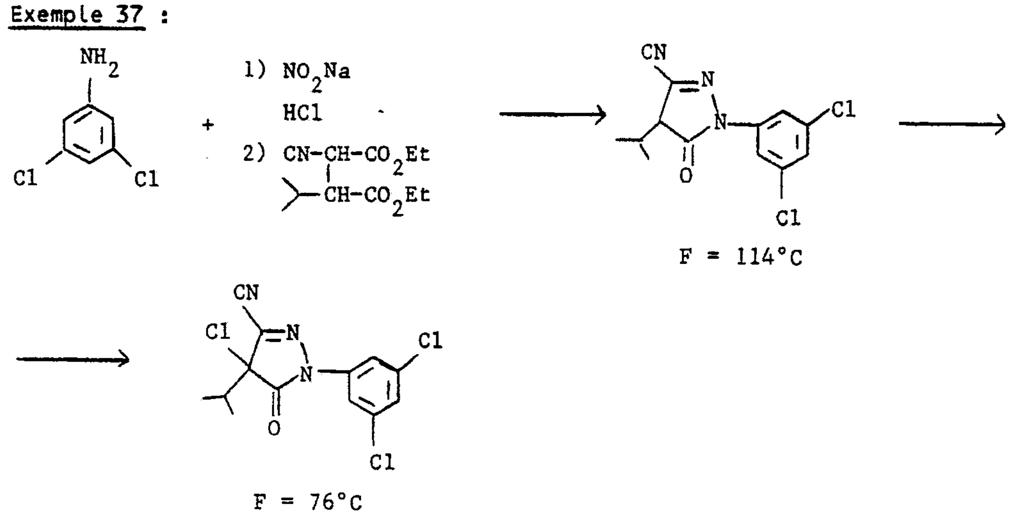


mode opératoire 4

Exemple 38 :

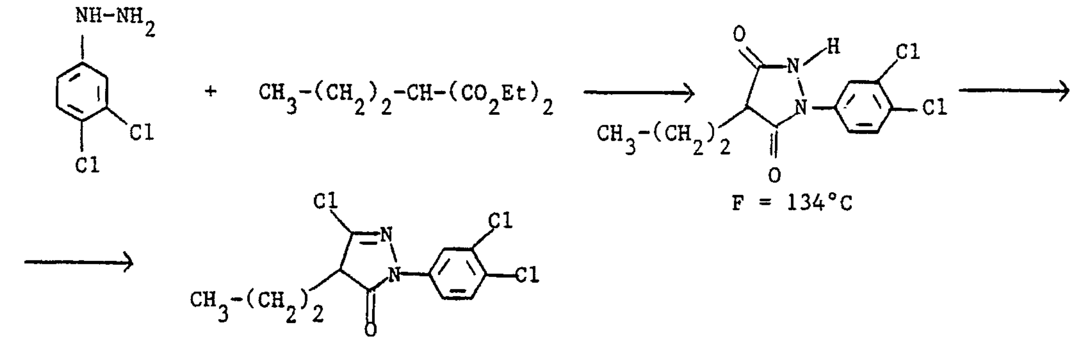


mode opératoire 3

Exemple 39 :

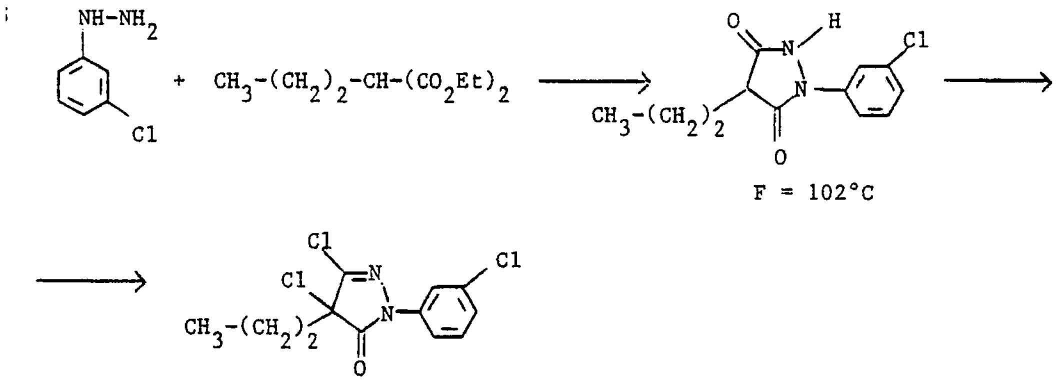


mode opératoire 3

Exemple 40 :

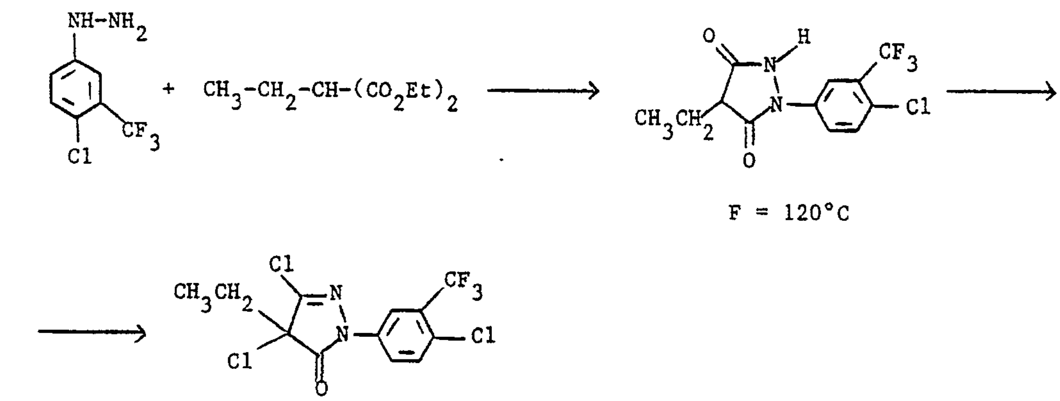


F = 50°C

mode opératoire 3

Exemple 41 :

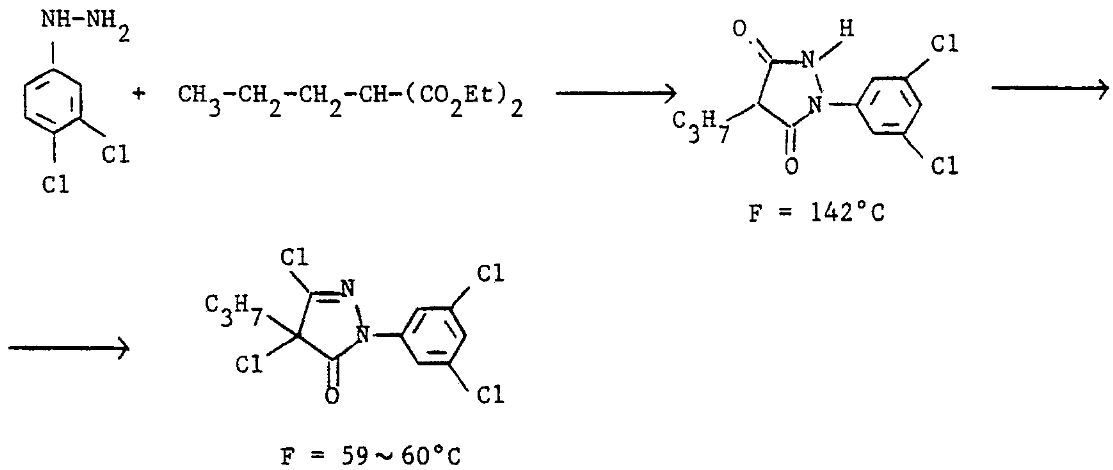


mode opératoire 3

Exemple 42 :

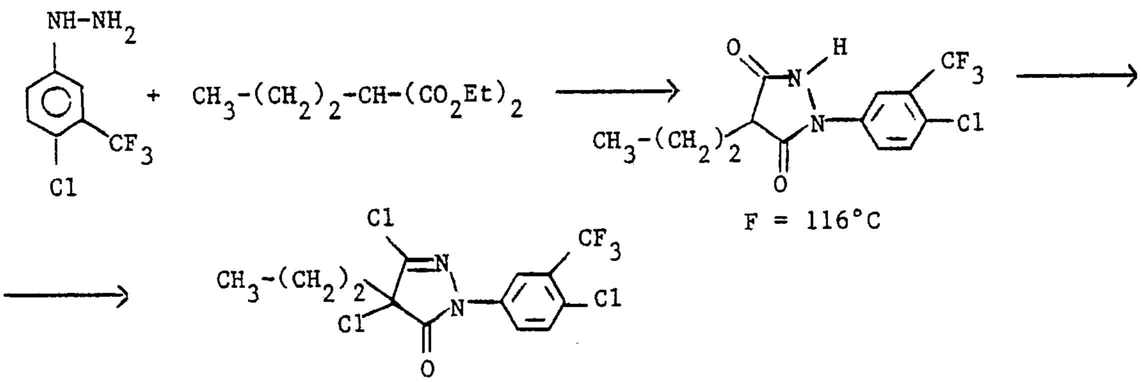


mode opératoire 3

**Exemple 43 : 1-(3,4-dichlorophényl) 3,4-dichloro 4-(1-méthylpropyl) 5-pyrazolone.**

**Stade A :** 1-(3,4-dichlorophényl) 4-(sec-butyl) pyrazolidine 3,5-dione.

On chauffe 10 heures à 180¤C (température extérieure) 50 g de 3,4-dichlorophénylhydrazine (85%) 122,5 g de sec-butyl malonate de diéthyle en distillant 21,3 g d'éthanol. On reprend dans 200 cm3 de chlorure de méthylène, extrait par la soude 2 N, puis à l'eau, réunit les phases aqueuses, lave au chlorure de méthylène, acidifie à pH 1 par de l'acide chlorhydrique concentré. On essore le précipité obtenu, le lave à l'eau, essore, sèche sous pression réduite à 40¤C et obtient 64,8 g de produit attendu. F = 118-120¤C.

**Stade B :** 1-(3,4-dichlorophényl) 3,4-dichloro 4-(1-méthylpropyl) 5-pyrazolone.

On porte 12 heures au reflux, 30 g du produit obtenu ci-dessus, 120 cm3 de oxychlorure de phospore et 72 g de pentachlorure de phosphore, refroidit, verse sur 2 kg de glace, extrait au chlorure de méthylène, lave à l'eau, sèche et amène à sec. On chromatographie sous pression le résidu sur silice (éluant : hexane-chlorure de méthylène 8-2) et recueille 4,6 g de produit attendu.

Spectre IR ($CDCl_3$) :
$>C=O$ : 1740 $cm^{-1}$
aromatique : 1593, 1562, 1478 $cm^{-1}$.

**Exemple 44 : 1-(3-chloro 4-bromophényl) 3-trifluorométhyl 4-allyl 4-hydroxy 5-pyrazolone.**

On refroidit à 0¤C, 1 g de 1-(3-chloro 4-bromophényl) 3-trifluorométhyl 4-allyl 5-pyrazolone dans 15 cm3 de chlorure de méthylène, ajoute 0,25 g de carbonate de sodium puis 0,53 g d'acide métachloroperbenzoïque et maintient à 0¤C pendant 3 heures et 30 minutes. On verse le mélange réactionnel dans de la glace additionnée d'hydrogénosulfite de sodium et extrait à l'acétate d'éthyle. On lave la phase organique avec une solution saturée en bicarbonate de sodium puis avec une solution de sulfate d'ammonium, enfin avec de l'eau saturée en chlorure de sodium, sèche et concentre à sec sous pression réduite. On chromatographie le résidu sur silice, élue au chlorure de méthylène et obtient 0,42 g de produit attendu. Après recristallisation dans un mélange chlorure de méthylène-éthanol le produit fond à 100,5¤C.

Spectre IR ($CDCl_3$) :
Présence OH : ~ 3560 $cm^{-1}$
+ associé ~ 3400 $cm^{-1}$

$>C=O$ complexe : max ~ 1748 $cm^{-1}$
: épaulement ~ 1720 $cm^{-1}$
$-CH=CH_2$ : C=C 1640 $cm^{-1}$
aromatique : 1616, 1589, 1562, 1474 $cm^{-1}$.

**Exemple 45 : 1-(3-chloro 4-bromophényl) 3-trifluorométhyl 4-fluoro 4-allyl 5-pyrazolone.**

On refroidit à -10¤C 4 g de 1-(3-chloro 4-bromophényl) 3-trifluorométhyl 4-allyl 4-hydroxy 5-pyrazolone dans 200 cm3 de chlorure de méthylène, ajoute en une fois 2,7 cm3 de diéthylaminosulfure trifluorure et agite 1 heure à -10¤C/-3¤C. On ajoute 120 cm3 d'une solution saturée en bicarbonate de sodium, agite 10 minutes, On décante, lave à l'eau,sèche et amène à sec. On chromatographie le résidu sur silice, élue par un mélange hexane-chlorure de méthylène (8-2) et obtient 1,1 g de produit attendu.

Spectre IR ($CDCl_3$ :
$>C=O$ : 1761 $cm^{-1}$
-C=C- : 1644 $cm^{-1}$
aromatique : 1614, 1590, 1568 $cm^{-1}$.

**Exemple 46 : 1-(3,5-dichlorophényl) 3-chloro 4-fluoro 4-méthyl 5-pyrazolone.**

**Stade A** : 1-(3,5-dichlorophényl) 3-chloro 4-hydroxy 4-méthyl 5-pyrazolone.

On refroidit à 0¤C, 1 g de 1-(3,5-dichlorophényl) 3-chloro 4-méthyl 5-pyrazolone, 30 cm3 d'acétate d'éthyle et 1 g de carbonate de potassium, ajoute 0,9 g d'acide métachloroperbenzoïque à 75% et agite 1 heure à 0¤C. On verse le mélange réactionnel dans de la glace additionnée d'hydrogénosulfite de sodium et extrait à l'acétate d'éthyle. Les phases organiques sont lavées avec une solution aqueuse saturée en bicarbonate de sodium, puis avec une solution de sulfate d'ammonium, enfin avec une solution aqueuse

saturée en chlorure de sodium. On les sèche et concentre à sec sous pression réduite. On dissout le résidu à chaud dans l'éther isopropylique, ajoute du pentane, refroidit et obtient 0,97 g de produit attendu.

Spectre IR :

OH : 3570 $cm^{-1}$
C=O : 1743 $cm^{-1}$
C=C : 1590 $cm^{-1}$
aromatique : 1570 $cm^{-1}$.

La 1-(3,5-dichlorophényl) 3-chloro 4-méthyl 5-pyrazolone utilisée au départ de l'exemple 46 a été préparée suivant le schéma ci-dessous :

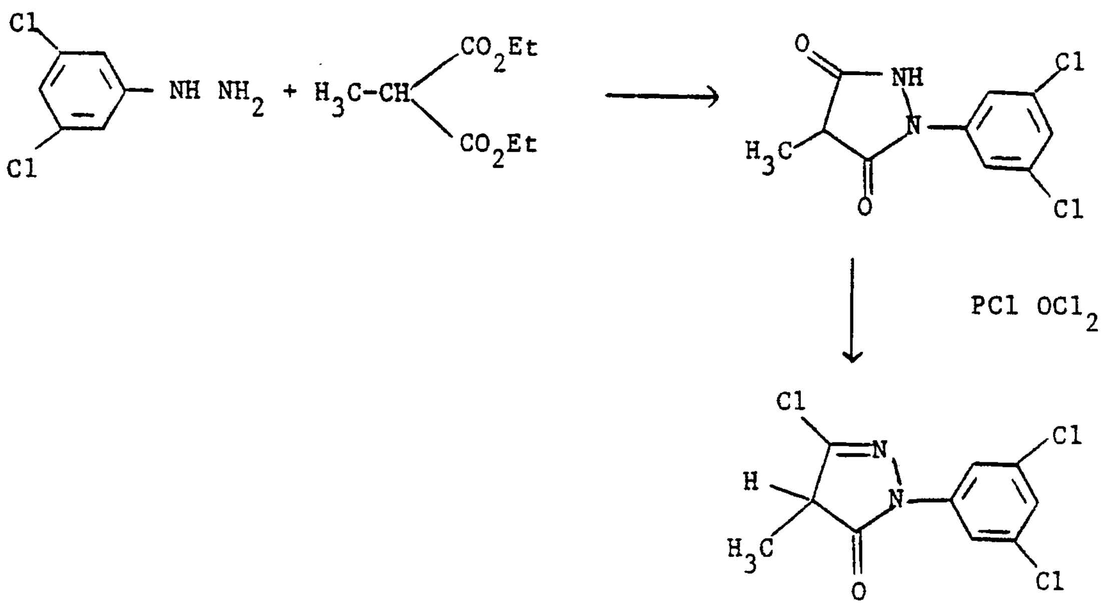


Analyse : $C_{10}H_7Cl_3N_2O$ : 277,54

| | | | | |
|---|---|---|---|---|
| Calculé : | C% 43,28 | H% 2,55 | Cl% 38,33 | N% 10,1 |
| Trouvé : | 43,6 | 2,5 | 38,1 | 10,1 |

Spectre IR :

$\nu$ C=C : 1600 $cm^{-1}$
aromatique : 1592, 1576, 1526 $cm^{-1}$.

Stade B : 1-(3,5-dichlorophényl) 3-chloro 4-fluoro 4-méthyl 5-pyrazolone.

On met en suspension 0,85 g de produit obtenu ci-dessus dans 15 cm3 de chlorure de méthylène et ajoute goutte à goutte 0,72 cm3 de diéthylamino sulfure trifluorure. Après 1 heure d'agitation à -5¤C, on verse le mélange réactionnel dans un mélange glace-bicarbonate de sodium, extrait au chlorure de méthylène, lave sucessivement avec des solutions de sulfate d'ammonium et de chlorure de sodium saturées, sèche et concentre à sec sous pression réduite. On chromatographie le résidu sur silice (éluant : cyclohexane-éther isopropylique 95-5) et récupère 0,3 g de produit attendu.

**Spectre IR** :
pas de OH
C=O : 1753 $cm^{-1}$
C=C : 1588 $cm^{-1}$
aromatique : 1570 $cm^{-1}$.

**Exemple 47 : 1-(3,5-dichlorophényl) 3-chloro 4-fluoro 4-(1-méthylpropyl) 5-pyrazolone.**

**Stade A** : 1-(3,5-dichlorophényl) 3-chloro 4-hydroxy 4-(1-méthylpropyl) 5-pyrazolone.

On refroidit à -20¤C, 0,7 g de 1-(3,5-dichlorophényl) 3-chloro 4-(1-méthylpropyl) 5-pyrazolone et 0,7 g de carbonate de potassium, introduit en une seule fois 0,7 g d'acide m-chloroperbenzoïque à 75%. On opère ensuite comme à l'exemple 46 et récupère 0,34 g de produit attendu utilisé tel quel pour la suite de la synthèse.

La 1-(3,5-dichlorophényl) 3-chloro 4-(1-méthylpropyl) 5-pyrazolone utilisée au départ de l'exemple 47 a été préparée suivant le schéma ci-dessous :

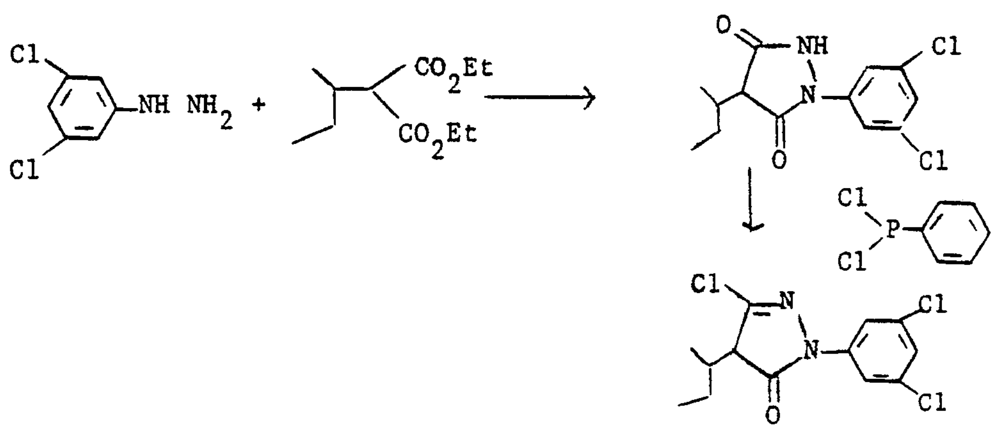

**Spectre IR** :
$\nu$ C=O : 1729 $cm^{-1}$
$\nu$ C=C : 1588 $cm^{-1}$
aromatique : 1567 $cm^{-1}$.

**Stade B** : 1-(3,5-dichlorophényl) 3-chloro 4-fluoro 4-(1-méthylpropyl) 5-pyrazolone.

On refroidit à -20¤C, 0,25 g du produit obtenu ci-dessus dans 20 cm3 de chlorure de méthylène, introduit 0,1 cm3 de diéthylaminosulfure trifluorure. Après 30 minutes d'agitation à -20¤C, on verse le milieu réactionnel dans un mélange glace et bicarbonate de sodium et extrait au chlorure de méthylène. On lave les phases organiques successivement avec des solutions saturées de sulfate d'ammonium et de chlorure de sodium, sèche et concentre à sec sous pression réduite. On chromatographie le résidu sur silice, élue par un mélange cyclohexane-éther isopropylique (95-5) et récupère 0,2 g de produit attendu.

Spectre RMN :

7,65 ppm : H aromatiques en 2 et 5

7,45 ppm : H aromatique en 4

3,5-3 ppm : $H_1$ du propyle.

**Exemple 48 : concentré émulsifiable selon l'inventeur.**

On a préparé une composition contenant en poids 15% de produit de l'exemple 1, 6,4% d'Atlox 4851 ® (triglycéride oxy éthylène combiné avec un sulfonate, indice d'acide 1,5) 3,2% d'Atlox 4855 ® (triglycéride oxyéthylèné combiné avec un sulfonate, indice d'acide 3) et 75,4% de xylène.

**Exemple 49 : poudre mouillable selon l'invention.**

On a préparé une poudre mouillable contenant 25% de composé de l'exemple 3, 15% d'Ekapersol ® - (produit de condensation du naphtalène sulfonate de sodium, 0,5% de Brecolane NVA ® (alcoyl naphtalène sulfonate de sodium) 34,5% de Zéozil 39 ® (silice hydratée synthétique obtenue par précipitation) et 25% de Vercoryl "S" ® (kaolin colloïdal).

**Exemple 50 : étude de l'activité herbicide de pré-émergence des composés de l'invention.**

Les végétaux utilisés sont cultivés en bac de culture (23x14x4 cm), à double fond, l'arrosage s'effectuant par dessous. Les espèces sont placées, à raison de 20 graines par espèces, en lignes espacées de 3 cm, dans un bac unique et il y a 4 essais pour chaque concentration. Les conditions de culture sont les suivantes : température : 20¤C ± 2¤C, humidité : 60% environ, éclairement : par tube fluorescent (lumière du jour + blanc brillant) de six heures à vingt-deux heures, chaque jour. Le mélange terreux utilisé est composé de 10 volumes de terre franche, de 10 volumes de sable de rivière et de 2 volumes de tourbe. Le traitement est effectué sur les parties aériennes après 21 jours de culture.

Le produit à étudier est appliqué, dans des conditions standard, à l'aide d'un micro-pulvérisateur, à dose de 2,5 Kg/ha et une dilution correspondant à 560 l/ha.

On effectue un essai avec un témoin non traité. Cet essai comporte le même nombre de plantules que les plantules traitées.

A la dose de 2,5 à 5 kg de matière active à l'hectare, il y a destruction des dicotylédones telles que le chenopode, l'amaranthe, le gaillet, la moutarde et des monocotylédones comme le vulpin, avec une selectivité sur les céréales.

## Revendications

1. Les composés de formule (I) :

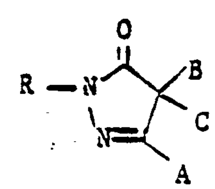

(I)

dans laquelle,

- R représente ou bien un radical phényle ou naphtyle, ou bien un radical thiazolyle, pyridinyle ou pyrimidyle, éventuellement substitué par un radical phényle, ou bien un radical

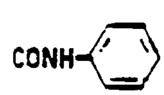

ou bien un radical benzyle, le radical R étant éventuellement substitué par un ou plusieurs radicaux choisis dans le groupe constitué par les atomes de fluor, de chlore, de brome, le radical $CF_3$, les radicaux alcoyles linéaires ou ramifiés, saturés ou insaturés renfermant jusqu'à 8 atomes de carbone et le radical OZ dans lequel Z représente un radical alcoyle linéaire ou ramifié, saturé ou insaturé renfermant jusqu'à 8 atomes de carbone ou un radical acyle renfermant jusqu'à 6 atomes de carbone,

- A représente un atome d'halogène, un radical $CF_3$ ou un radical C≡N,
- B et C identiques ou différents représentent soit un atome d'hydrogène, soit un atome d'halogène, soit un radical hydroxy, soit un radical OR' dans lequel R' représente un radical alcoyle linéaire ou ramifié, saturé ou insaturé renfermant jusqu'à 8 atomes de carbone, un radical acyle renfermant jusqu'à 6 atomes de carbone, un radical alkylsulfonyl renfermant jusqu'à 8 atomes de carbone ou un radical benzylsulfonyl, soit un radical benzyle éventuellement substitué par un ou plusieurs radicaux choisis dans le groupe constitué par les atomes de fluor, de chlore, de brome, le radical $CF_3$ et les radicaux alcoyles linéaires ou ramifiés, saturés ou insaturés renfermant jusqu'à 8 atomes de carbone, soit un radical alcoyle renfermant de 1 à 8 atomes de carbone linéaire ou ramifié, saturé ou insaturé, éventuellement interrompu par un ou plusieurs atomes d'oxygène et éventuellement substitué par un ou plusieurs atomes d'halogène, étant entendu que B et C ne peuvent pas représenter en même temps soit un atome de fluor, soit un radical hydroxy, et que B et C ne peuvent pas représenter en même temps un atome d'hydrogène, lorsque A représente un radical CN et R un radical 3-chlorophényle.

**2.** Les composés de formule I dans lesquels R représente un radical phényle éventuellement substitué par un ou plusieurs atomes de chlore ou de brome.

**3.** Les composés de formule I tels que définis à la revendication 1 ou 2 caractérisés en ce que A représente un atome de chlore.

**4.** Les composés de formule I tels que définis à la revendication 1 ou 2 caractérisés en ce que A représente un radical $CF_3$.

**5.** Les composés de formule I tels que définis à la revendication 1 ou 2 caractérisés en ce que A représente un radical C≡N.

**6.** Les composés de formule I tels que définis à l'une quelconque des revendications 1 à 5 caractérisés en ce que les radicaux B et C identiques ou différents représentent un atome d'hydrogène, un atome de chlore ou de brome, un radical alcoyle saturé linéaire ou ramifié renfermant jusqu'à 6 atomes de carbone ou un radical allyle.

**7.** Le composé répondant à la formule (I) dont le nom suit :
- la 1-(3,4-dichlorophényl) 3,4-dichloro 4-(2-méthylpropyl) 5-pyrazolone.

**8.** Les composés répondant à la formule (I) dont les noms suivent :
- la 1-(3-chloro 4-bromophényl) 3-trifluorométhyl 4-chloro 4-méthyl 5-pyrazolone,
- la 1-(3,5-dichlorophényl) 3,4-dichloro 4-méthyl 5-pyrazolone,
- la 1-(3,4-dichlorophényl) 3,4-dichloro 4-n-propyle 5-pyrazolone.

**9.** Procédé de préparation des composés de formule (I) tels que définis à l'une quelconque des revendications 1 à 8 caractérisé en ce que l'on soumet un composé de formule (II) :

$H_2N$-NH-R (II)

dans laquelle R conserve la même signification que précédemment à l'action d'un composé de formule III

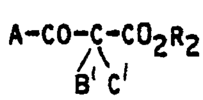

(III)

dans laquelle A conserve sa signification précédente et B′ et C′ ayant la signification indiquée pour B et C à l'exception du radical OH ou OR′ et $R_2$ représente un radical alcoyle renfermant jusqu'à 8 atomes de carbone pour obtenir le composé de formule ($I_A$) :

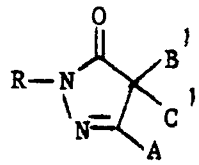

($I_A$)

dans laquelle R, A, B′ et C′ ont la signification indiquée ci-dessus que l'on soumet éventuellement si l'un ou l'autre (ou l'un et l'autre) des radicaux B′ et C′ représente un atome d'hydrogène, soit à l'action d'un agent d'halogénation pour obtenir le dérivé de formule (I) correspondant dans lequel B′ ou C′ ou B′ et C′ représentent un atome d'halogène, soit à l'action d'un agent d'oxydation pour obtenir le dérivé de formule (I) correspondant dans lequel B′ ou C′ représente un radical hydroxy, produit que l'on soumet, si désiré, soit à l'action d'un agent capable d'introduire un radical alkyle, acyle, benzylsulfonyle, alkylsulfonyle, soit à l'action d'un agent d'halogénation pour obtenir le produit de formule (I) correspondant.

**10.** Variante du procédé de préparation selon la revendication 9, caractérisée en ce que l'on soumet le composé de formule (II) :

$H_2N$-NHR (II)

dans laquelle R conserve sa signification précédente, à l'action du composé de formule (IV) :

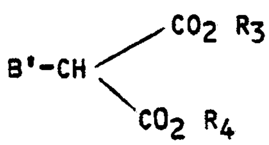

(IV)

dans laquelle B′ conserve sa signification précédente, $R_3$ et $R_4$ identiques ou différents représentent un radical alcoyle renfermant jusqu'à 6 atomes de carbone pour obtenir le composé de formule (V) :

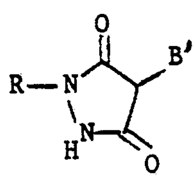

(V)

que l'on soumet à l'action d'un agent de chloruration pour obtenir

- si B′ ne représente pas un atome d'hydrogène, le composé de formule ($I_B$) ou ($I_C$) :

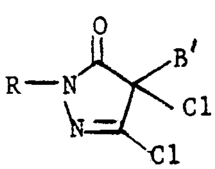

($I_B$) ou

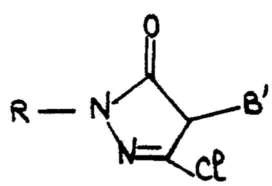

($I_C$)

- et si B′ représente un atome d'hydrogène le composé de formule ($I_D$) :

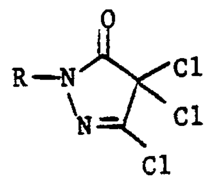

($I_D$)

puis soumet si désiré le produit de formule ($I_C$) à l'action du chlore pour obtenir le produit de formule ($I_D$) correspondant ou bien à l'action d'un agent d'oxydation pour obtenir le produit un formule ($I_E$) :

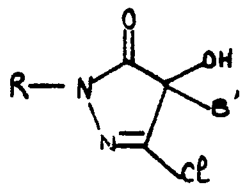

($I_E$)

que l'on soumet si désiré soit à l'action d'un agent capable d'introduire un radical alkyle, acyle, alkylsulfonyle ou benzylsulfonyle, soit à l'action d'un agent d'halogénation pour obtenir le produit de formule (I) correspondant.

**11.** Variante du procédé selon la revendication 9, caractérisée en ce que l'on soumet un composé de formule (VI) :

$RNH_2$ (VI)

dans laquelle R conserve la même signification que précédemment, à l'action d'un agent de diazotation, pour obtenir le sel de diazonium correspondant que l'on soumet à l'action du composé de formule (VII) :

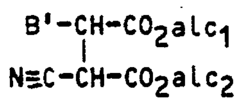

(VII)

dans laquelle B′ conserve la même signification que précédemment, $alc_1$ et $alc_2$, identiques ou différents représentent un radical alcoyle renfermant jusqu'à 8 atomes de carbone pour obtenir le composé de formule ($I_F$) :

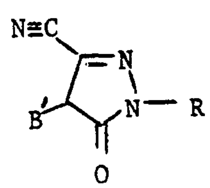

($I_F$)

que l'on soumet éventuellement à l'action d'un agent d'halogénation pour obtenir si B′ représente un atome d'hydrogène, le composé dihalogéné de formule ($I_G$) :

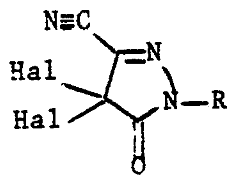

$(I_G)$

et si B' ne représente pas un atome d'hydrogène, le composé monohalogéné correspondant ($I_H$) :

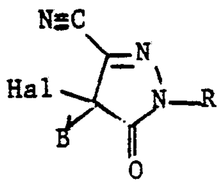

$(I_H)$

soit à l'action d'un agent d'oxydation pour obtenir un produit de formule ($I_I$) :

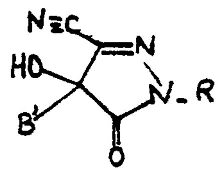

$(I_I)$

que l'on soumet si désiré soit à l'action d'un agent capable d'introduire un radical alkyle, acyle, alkylsulfonyle ou benzylsulfonyle soit à l'action d'un agent d'halogénation pour obtenir le produit de formule (I) correspondant.

**12.** Les compositions herbicides caractérisées en ce qu'elles contiennent comme principe actif au moins un des composés de formule (I) tels que définis à la revendication 1.

**13.** Les compositions herbicides caractérisées en ce qu'elles contiennent comme principe actif au moins un des composés de formule (I) tels que définis à l'une quelconque des revendications 2 à 6.

**14.** Les compositions herbicides caractérisées en ce qu'elles contiennent comme principe actif au moins un des composés de formule (I) tels que définis à la revendication 7 ou 8.

## Claims

**1.** The compounds of formula (I):

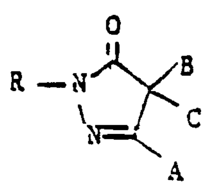

(I)

in which

- R represents either a phenyl or naphthyl radical, or a thiazolyl, pyridinyl or pyrimidyl radical, optionally substituted by a phenyl radical, or a

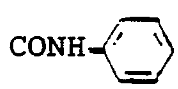

radical or a benzyl radical, the R radical being optionally substituted by one or more radicals chosen from the group constituted by fluorine, chlorine and bromine atoms, the $CF_3$ radical,

saturated or unsaturated, linear or branched alkyl radicals containing up to 8 carbon atoms and the OZ radical in which Z represents a saturated or unsaturated, linear or branched alkyl radical containing up to 8 carbon atoms or an acyl radical containing up to 6 carbon atoms,

- A represents a halogen atom, a $CF_3$ radical or a C≡N radical,
- B and C, identical or different, represent either a hydrogen atom, or a halogen atom, or a hydroxy radical, or an OR' radical in which R' represents a saturated or unsaturated, linear or branched alkyl radical containing up to 8 carbon atoms, an acyl radical containing up to 6 carbon atoms, an alkylsulphonyl radical containing up to 8 carbon atoms or a benzylsulphonyl radical, or a benzyl radical optionally substituted by one or more radicals chosen from the group constituted by fluorine, chlorine and bromine atoms, the $CF_3$ radical and saturated or unsaturated, linear or branched alkyl radicals containing up to 8 carbon atoms, or a saturated or unsaturated, linear or branched alkyl radical containing 1 to 8 carbon atoms, optionally interrupted by one or more oxygen atoms and optionally substituted by one or more halogen atoms, it being understood that B and C cannot represent at the same time either a fluorine atom or a hydroxy radical, and that B and C cannot represent at the same time a hydrogen atom, when A represents a CN radical and R a 3-chlorophenyl radical.

**2.** The compounds of formula (I) in which R represents a phenyl radical optionally substituted by one or more chlorine or bromine atoms.

**3.** The compounds of formula (I) as defined in claim 1 or 2, characterized in that A represents a chlorine atom.

**4.** The compounds of formula (I) as defined in claim 1 or 2, characterized in that A represents a $CF_3$ radical.

**5.** The compounds of formula (I) as defined in claim 1 or 2, characterized in that A represents a C≡N radical.

**6.** The compounds of formula (I) as defined in any one of claims 1 to 5, characterized in that the radicals B and C, identical or different, represent a hydrogen atom, a chlorine or bromine atom, a saturated linear or branched alkyl radical containing up to 6 carbon atoms or an allyl radical.

**7.** The compound corresponding to formula (I) of which the name follows:

- 1-(3,4-dichlorophenyl)-3,4-dichloro-4'-(2-methylpropyl)-5-pyrazolone.

**8.** The compounds corresponding to formula (I) of which the names follow:

- 1-(3-chloro-4-bromophenyl)-3-trifluoromethyl-4-chloro-4-methyl-5-pyrazolone,
- 1-(3,5-dichlorophenyl)-3,4-dichloro-4-methyl-5-pyrazolone,
- 1-(3,4-dichlorophenyl)-3,4-dichloro-4-n-propyl-5-pyrazolone.

**9.** Preparation process for the compounds of formula (I) as defined in any one of claims 1 to 8, characterized in that a compound of formula (II):

$H_2N-NH-R$ (II)

in which R keeps the same meaning as previously, is subjected to the action of a compound of formula (III):

$$A-CO-\underset{B'\ \ C'}{\overset{}{C}}-CO_2R_2 \qquad (III)$$

in which A keeps its previous meaning and B' and C' have the meaning indicated for B and C except for the OH or OR' radical, and $R_2$ represents an alkyl radical containing up to 8 carbon atoms, in order to obtain the compound of formula ($I_A$):

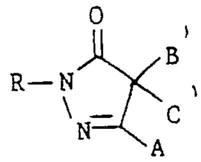

($I_A$)

in which R, A, B' and C' have the meaning indicated above, which is optionally subjected, if one or other (or both) of the B' and C' radicals represents a hydrogen atom, either to the action of a halogenation agent to obtain the corresponding derivative of formula (I) in which B' or C' or B' and C' represent a halogen atom, or to the action of an oxidizing agent to obtain the corresponding derivative of formula (I) in which B' or C' represents a hydroxy radical, which product is subjected, if desired, either to the action of an agent which can introduce an alkyl, acyl, arylsulphonyl, or alkylsulphonyl radical, or to the action of a halogenation agent to obtain the corresponding product of formula (I).

**10.** Variation of the preparation process according to claim 9, characterized in that the compound of formula (II):

$H_2N$-NHR (II)

in which R keeps its previous meaning, is subjected to the action of the compound of formula (IV):

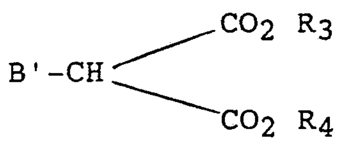

(IV)

in which B' keeps its previous meaning, $R_3$ and $R_4$, identical or different, represent an alkyl radical containing up to 6 carbon atoms, in order to obtain the compound of formula (V):

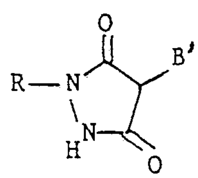

(V)

which is subjected to the action of a chlorination agent in order to obtain

- if B' does not represent a hydrogen atom, the compound of formula ($I_B$) or ($I_C$):

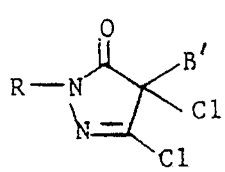

($I_B$) or

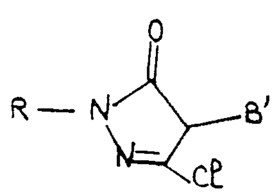

($I_C$)

- and if B' represents a hydrogen atom, the compound of formula ($I_D$):

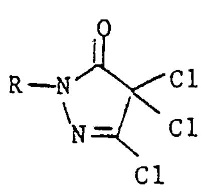

($I_D$)

then, if desired, the product of formula ($I_C$) is subjected to the action of chlorine to obtain the corresponding product of formula ($I_D$), or to the action of an oxidizing agent to obtain the product of formula ($I_E$):

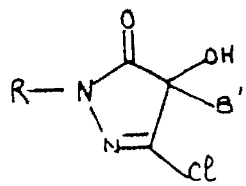

($I_E$)

which is subjected, if desired, either to the action of an agent which can introduce an alkyl, acyl, alkylsulphonyl or arylsulphonyl radical, or to the action of a halogenation agent to obtain the corresponding product of formula (I).

**11.** Variation of the process according to claim 9, characterized in that a compound of formula (VI):

$RNH_2$ (VI)

in which R keeps the same meaning as previously, is subjected to the action of a diazotization agent, to obtain the corresponding diazonium salt which is subjected to the action of a compound of formula (VII):

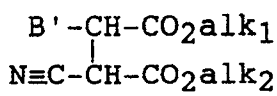

(VII)

in which B' keeps the same meaning as previously, $alk_1$ and $alk_2$, identical or different, represent an alkyl radical containing up to 8 carbon atoms, to obtain the compound of formula ($I_F$):

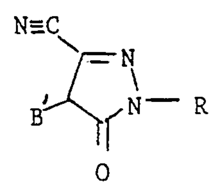

($I_F$)

which is optionally subjected to the action of a halogenation agent to obtain, if B' represents a hydrogen atom, the dihalogenated compound of formula ($I_G$):

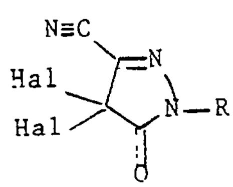

($I_G$)

and if B' does not represent a hydrogen atom, the corresponding monohalogenated compound ($I_H$):

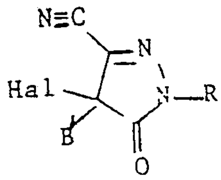

($I_H$)

or to the action of an oxidizing agent to obtain a product of formula ($I_I$):

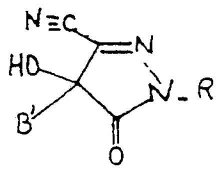

($I_I$)

which is subjected, if desired, either to the action of an agent which can introduce an alkyl, acyl, alkylsulphonyl or arylsulphonyl radical, or to the action of a halogenation agent to obtain the corresponding product of formula (I).

**12.** Herbicide compositions characterized in that they contain as active ingredient at least one of the compounds of formula (I) as defined in claim 1.

**13.** Herbicide compositions characterized in that they contain as active ingredient at least one of the compounds of formula (I) as defined in any one of claims 2 to 6.

**14.** Herbicide compositions characterized in that they contain as active ingredient at least one of the compounds of formula (I) as defined in claim 7 or 8.

**Ansprüche**

**1.** Verbindungen der Formel (I)

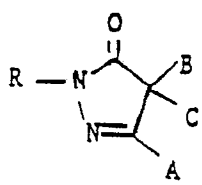

(I)

worin

- R entweder einen Phenyl- oder Napthylrest oder einen Thiazolyl-, Pyridinyl- oder Pyrimidylrest, der gegebenenfalls durch einen Phenylrest substituiert ist, oder einen Rest

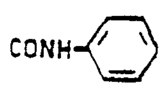

oder einen Benzylrest bedeutet, wobei der Rest R gegebenenfalls durch einen oder mehrere Reste, ausgewählt unter den Fluor-, Chlor- und Bromatomen, der $CF_3$ -Gruppe, den gesättigten oder ungesättigten, linearen oder verzweigten Alkylresten mit bis zu 8 Kohlenstoffatomen und dem Rest OZ, worin Z für eine gesättigte oder ungesättigte, lineare oder verzweigte Alkylgruppe mit bis zu 8 Kohlenstoffatomen oder einen Acylrest mit bis zu 6 Kohlenstoffatomen steht, substituiert ist,
- A für ein Halogenatom, eine $CF_3$ -Gruppe oder eine Gruppe C≡N steht,

- B und C, die gleich oder voneinander verschieden sind, entweder ein Wasserstoffatom oder ein Halogenatom oder eine Hydroxygruppe oder einen Rest OR', worin R' für einen gesättigten oder ungesättigten, linearen oder verzweigten Alkylrest mit bis zu 8 Kohlenstoffatomen, einen Acylrest mit bis zu 6 Kohlenstoffatomen, einen Alkylsulfonylrest mit bis zu 8 Kohlenstoffatomen oder einen Benzylsulfonylrest steht, oder einen Benzylrest, der gegebenenfalls durch einen oder mehrere Reste, ausgewählt unter den Fluor-, Chlor- und Bromatomen, der $CF_3$ -Gruppe und den gesättigten oder ungesättigten, linearen oder verzweigten Alkylresten mit bis zu 8 Kohlenstoffatomen, substituiert ist, oder einen gesättigten oder ungesättigten, linearen oder verzweigten Alkylrest mit 1 bis 8 Kohlenstoffatomen, der gegebenenfalls durch ein oder mehrere Sauerstoffatome unterbrochen und gegebenenfalls durch ein oder mehrere Halogenatome substituiert ist, bedeuten mit der Maßgabe, daß B und C nicht gleichzeitig entweder ein Fluoratom oder eine Hydroxygruppe wiedergeben können, und daß B und C nicht gleichzeitig ein Wasserstoffatom bedeuten können, wenn A einen Rest CN wiedergibt, und R für einen 3-Chlorphenylrest steht.

2. Verbindungen der Formel (I), worin R eine Phenylgruppe bedeutet, die gegebenenfalls durch ein oder mehrere Chlor- oder Bromatome substituiert ist.

3. Verbindungen der Formel (I) gemäß Anspruch 1 oder 2, dadurch gekennzeichnet, daß A für ein Chloratom steht.

4. Verbindungen der Formel (I) gemäß Anspruch 1 oder 2, dadurch gekennzeichnet, daß A für eine $CF_3$ -Gruppe steht.

5. Verbindungen der Formel (I) gemäß Anspruch 1 oder 2, dadurch gekennzeichnet, daß A für eine Gruppe C≡N steht.

6. Verbindungen der Formel (I) gemäß einem der Ansprüche 1 bis 5, dadurch gekennzeichnet, daß die Reste B und C, die gleich oder voneinander verschieden sind, ein Wasserstoffatom, ein Chloratom oder ein Bromatom, eine lineare oder verzweigte, gesättigte Alkylgruppe mit bis zu 6 Kohlenstoffatomen oder einen Allylrest wiedergeben.

7. Verbindung der Formel (I) mit der folgenden Bezeichnung:
1-(3,4-Dichlorphenyl)-3,4-dichlor-4 -(2-methylpropyl)-5-pyrazolon.

8. Verbindungen der Formel (I) mit den folgenden Bezeichnungen:
1-(3-Chlor-4-Bromphenyl)-3-trifluormethyl-4-chlor-4-methyl-5-pyrazolon,
1-(3,5-Dichlorphenyl)-3,4-dichlor-4-methyl-5-pyrazolon,
1-(3,4-Dichlorphenyl)-3,4-dichlor-4-n-propyl-5-pyrazolon.

9. Verfahren zur Herstellung der Verbindungen der Formel (I) gemäß einem der Ansprüche 1 bis 8, dadurch gekennzeichnet, daß man eine Verbindung der Formel (II)

$H_2N-NH-R$ (II)

worin R die vorstehend angegebene Bedeutung besitzt, der Einwirkung einer Verbindung der Formel (III)

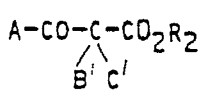

(III)

worin A die vorstehend angegebene Bedeutung besitzt, und B' und C' die für B und C angegebene Bedeutung mit Ausnahme des Restes OH oder OR' besitzen, und $R_2$ für eine Alkylgruppe mit bis zu 8 Kohlenstoffatomen steht, unterzieht, um zu der Verbindung der Formel ($I_A$)

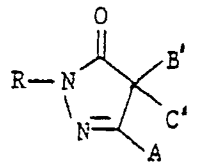

$(I_A)$

worin R, A, B' und C' die angegebene Bedeutung besitzen zu gelangen, welche man gegebenenfalls, wenn der eine oder andere (oder der eine und der andere) der Reste B' und C' für ein Wasserstoffatom steht, entweder der Einwirkung eines Halogenierungsmittels unterzieht, um zu dem entsprechenden Derivat der Formel (I) zu gelangen, worin B' oder C' oder B' und C' für ein Halogenatom stehen, oder der Einwirkung eines Oxidationsmittels unterzieht, um zu dem entsprechenden Derivat der Formel (I) zu gelangen, worin B' oder C' für eine Hydroxygruppe steht, welches Produkt man gewünschtenfalls entweder der Einwirkung eines Mittels unterzieht, das befähigt ist, eine Alkyl-, Acyl-, Arylsulfonyl- oder Alkylsulfonylgruppe einzuführen, oder der Einwirkung eines Halogenierungsmittels unterzieht, um das entsprechende Produkt der Formel (I) zu erhalten.

**10.** Abänderung des Verfahrens gemäß Anspruch 9, dadurch gekennzeichnet, daß man die Verbindung der Formel (II)

$H_2N\text{-}NHR$ (II)

worin R die vorstehend angegebene Bedeutung besitzt, der Einwirkung der Verbindung der Formel (IV)

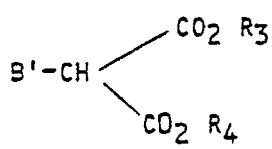

(IV)

worin B' die vorstehend angegebene Bedeutung besitzt, $R_3$ und $R_4$ gleich oder voneinander verschieden sind, einen Alkylrest mit bis zu 6 Kohlenstoffatomen bedeutet, unterzieht, um zu der Verbindung der Formel (V)

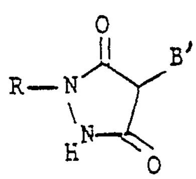

(V)

zu gelangen, welche man der Einwirkung eines Chlorierungsmittels unterzieht, um

- wenn B' kein Wasserstoffatom bedeutet, die Verbindung der Formel ($I_B$) oder ($I_C$)

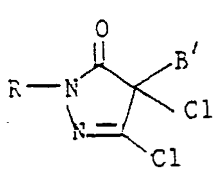

($I_B$) oder

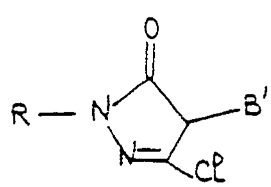

($I_C$)

- und wenn B' ein Wasserstoffatom bedeutet, die Verbindung der Formel ($I_D$)

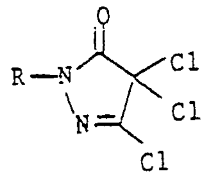

$(I_D)$

zu erhalten, hiernach gewünschtenfalls das Produkt der Formel ($I_C$) der Einwirkung von Chlor unterzieht, um zu dem entsprechenden Produkt der Formel ($I_D$) zu gelangen, oder der Einwirkung eines Oxidationsmittels unterzieht, um zu dem Produkt der Formel ($I_E$)

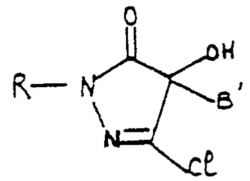

$(I_E)$

zu gelangen, welches man gewünschtenfalls entweder der Einwirkung eines Mittels, das befähigt ist, einen Alkyl-, Acyl-, Alkylsulfonyl- oder Arylsulfonylrest einzuführen, oder der Einwirkung eines Halogenierungsmittels unterzieht, um zu dem entsprechenden Produkt der Formel (I) zu gelangen.

**11.** Abänderung des Verfahrens gemäß Anspruch 9, dadurch gekennzeichnet, daß man eine Verbindung der Formel (VI)

$RNH_2$ (VI)

worin R die vorstehende Bedeutung besitzt, der Einwirkung eines Diazotierungsmittels unterzieht, um zu dem entsprechenden Diazoniumsalz zu gelangen, welches man der Einwirkung der Verbindung der Formel (VII)

$$\begin{array}{l} B'\text{-}CH\text{-}CO_2\,alc_1 \\ N{\equiv}C\text{-}CH\text{-}CO_2\,alc_2 \end{array} \qquad (VII)$$

worin B' die vorstehend angegebene Bedeutung besitzt, $alc_1$ und $alc_2$, die gleich oder voneinander verschieden sind, für eine Alkylgruppe mit bis zu 8 Kohlenstoffatomen stehen, unterzieht, um zu der Verbindung der Formel ($I_F$)

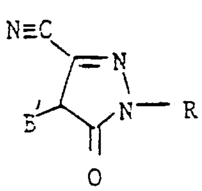

$(I_F)$

zu gelangen, welche man gegebenenfalls der Einwirkung eines Halogenierungsmittels unterzieht, um, wenn B' für ein Wasserstoffatom steht, die Dihalogenverbindung der Formel ($I_G$)

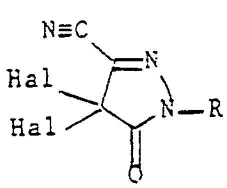

$(I_G)$

und wenn B' nicht für ein Wasserstoffatom steht, die entsprechende Monohalogenverbindung ($I_H$)

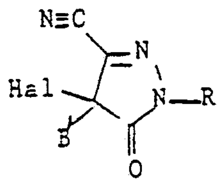

($I_H$)

zu erhalten, oder der Einwirkung eines Oxidationsmittels unterzieht, um zu einem Produkt der Formel ($I_I$)

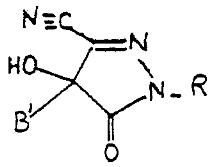

($I_I$)

zu gelangen, welches man gewünschtenfalls entweder der Einwirkung eines Mittels unterzieht, das befähigt ist, einen Alkyl-, Acyl-, Alkylsulfonyl- oder Arylsulfonylrest einzuführen, oder der Einwirkung eines Halogenierungsmittels unterzieht, um zu dem entsprechenden Produkt der Formel (I) zu gelangen.

**12.** Herbizide Zusammensetzungen, dadurch gekennzeichnet, daß sie als Wirkstoff zumindest eine der Verbindungen der Formel (I) gemäß Anspruch 1 enthalten.

**13.** Herbizide Zusammensetzungen, dadurch gekennzeichnet, daß sie als Wirkstoff zumindest eine der Verbindungen der Formel (I) gemäß einem der Ansprüche 2 bis 6 enthalten.

**14.** Herbizide Zusammensetzungen, dadurch gekennzeichnet, daß sie als Wirkstoff zumindest eine der Verbindungen der Formel (I) gemäß Anspruch 7 oder 8 enthalten.